# EUROPEAN PATENT APPLICATION

(11) **EP 4 840 863 A1**
(43) Date of publication of application: **18.11.2026**
(21) Application number: 25738614.4
(22) Date of filing: 13.01.2025
(51) Int. Cl.: C07D 493/04, C07D 493/10, A61P 35/00, A61K 31/365, A61K 31/381, A61K 31/4525

(54) **FLUORINE-CONTAINING PARTHENOLIDE DERIVATIVE, PREPARATION METHOD THEREFOR AND USE THEREOF**

(30) Priority: 12.01.2024 CN 202410049665
(71) Applicant: Accendatech Luoyang Co., Ltd., Luoyang, Henan 471003 (CN); Accendatech Co. Ltd., Tianjin 300102 (CN)
(72) Inventor: DUAN, Xiyan, Luoyang, Henan 471003 (CN); WANG, Junqi, Luoyang, Henan 471003 (CN); ZHANG, Xuemei, Luoyang, Henan 471003 (CN); HUO, Xiaoguang, Luoyang, Henan 471003 (CN); SHEN, Zhuo, Luoyang, Henan 471003 (CN); BAI, Cuigai, Luoyang, Henan 471003 (CN); LI, Hongbo, Luoyang, Henan 471003 (CN); QIU, Chuanjiang, Luoyang, Henan 471003 (CN)
(74) Representative: Gille Hrabal Partnerschaftsgesellschaft mbB Patentanwälte
(86) International application number: PCT/CN2025/072153
(87) International publication number: WO 2025/149087

(57) **Abstract**

The present invention provides a fluorine-containing parthenolide derivative compound as shown in formula (I), and a deuterated form, tautomer, stereoisomer, hydrate, solvate, pharmaceutically acceptable salt or prodrug thereof, which have good tumor inhibitory activity, and can be used for the prevention and/or treatment of cancer. The preparation method provided by the present invention is simple to operate and can efficiently and rapidly construct the fluorine-containing parthenolide molecules.

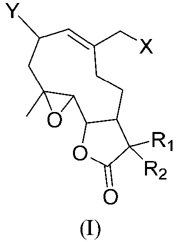

## Description

This application claims the benefit of priority from an earlier application filed by the applicant with the China National Intellectual Property Administration on January 12, 2024, with the patent application number 202410049665.5 and entitled "FLUORINE-CONTAINING PARTHENOLIDE DERIVATIVE, PREPARATION METHOD THEREFOR AND USE THEREOF". The entire content of the earlier application is incorporated herein by reference.

### TECHNICAL FIELD

The present disclosure belongs to the field of pharmaceutical technology, and specifically relates to fluorine-containing parthenolide derivatives, preparation methods and uses thereof.

### BACKGROUND

Parthenolide ((-)-Parthenolide, abbreviated as PTL) is a natural sesquiterpene lactone and a secondary metabolite of the Asteraceae and Magnoliaceae families. Initially, this class of compounds was first isolated from the Western plant feverfew in 1965, and was later found to be present in higher amounts in Magnolia. Parthenolide has excellent anti-inflammatory and anti-tumor activities. More importantly, parthenolide is active against tumor cells while having almost no effect on normal cells. Furthermore, parthenolide is the first small-molecule drug discovered to selectively induce apoptosis in cancer stem cells.

The potential mechanism of the anti-inflammatory effect of parthenolide may be to attenuate the production of inflammatory mediators by inhibiting the activation of the Akt, mTOR, and NF-κB pathways mediated by toll-like receptor 4. Recent studies have shown that parthenolide may act as a potential drug to induce apoptosis in various types of human cancer cells, including colorectal cancer (CRC), chronic myeloid leukemia (CML), pancreatic cancer, osteosarcoma, and breast cancer. Parthenolide not only induces apoptosis in tumor cells but also has an antiproliferative effect on tumor cells. For example, parthenolide may inhibit lung cancer growth by suppressing the IGF-1R-mediated PI3K/Akt/FoxO3α signaling pathway and inhibit the development of non-small cell lung cancer cells via the B-Raf/MAPK/Erk pathway. In addition, parthenolide can inhibit colorectal cancer cell growth by suppressing ubiquitin-specific peptidase 7 (USP7/Wnt) signaling. Despite its unique biological activities, poor water solubility and structural instability limit the clinical application of parthenolide. The Michael addition product of parthenolide can not only overcome the drawback of poor water solubility but also increase the bioavailability of the drug. For example, the dimethylamine addition product of parthenolide (DMAPT) has entered clinical trials for cancer treatment. There is a need to further develop parthenolide-like compounds with medicinal value that are easy to synthesize and produce.

### SUMMARY

The present disclosure provides a compound of formula (I), a deuterated form, tautomer, stereoisomer, hydrate, solvate, pharmaceutically acceptable salt, or prodrug thereof: 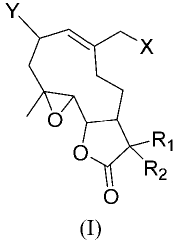 wherein:
X is selected from the following groups which are unsubstituted or optionally substituted with one, two, or more Rₓ: OH, SH, C₁₋₁₀ alkyl;
Y is selected from H, and the following groups which are unsubstituted or optionally substituted with one, two, or more R_{y}: OH, SH, C₁₋₁₀ alkyl;
each Rₓ and R_{y} is the same or different and is independently selected from H, OH, deuterium, halogen, CN, C₁₋₁₀ alkyl, C₁₋₁₀ alkoxy, halo-C₁₋₁₀ alkyl, halo-C₁₋₁₀ alkoxy, C₃₋₁₀ cycloalkyl, 3-10 membered heterocyclyl, C₆₋₁₄ aryl, 5-14 membered heteroaryl;
R₁ is selected from hydrogen and deuterium;
R₂ is selected from the following group which is unsubstituted or optionally substituted with one, two, or more
R₂₁: 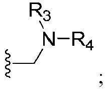 or 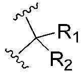 is selelcted from the following group which is unsubstituted or optionally substituted with one or two R₂₂: 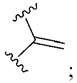
R₃ and R₄ are the same or different and are each independently selected from H, and the following groups which are unsubstituted or optionally substituted with one, two, or more R₃₁: C₁₋₁₀ alkyl, C₃₋₁₀ cycloalkyl, 3-10 membered heterocyclyl, C₆₋₁₄ aryl, 5-14 membered heteroaryl; or, R₃ and R₄ together with the N atom to which they are attached form a 3-10 membered heterocyclyl which is unsubstituted or optionally substituted with one, two, or more R₃₂;
each R₂₁, R₂₂, R₃₁, and R₃₂ is the same or different and is independently selected from H, OH, deuterium, halogen, CN, C₁₋₁₀ alkyl, C₃₋₁₀ cycloalkyl, 3-10 membered heterocyclyl, C₆₋₁₄ aryl, and 5-14 membered heteroaryl.

According to an embodiment of the present disclosure, X is selected from the group consisting of the following groups which are unsubstituted or optionally substituted with one, two, or more Rₓ: OH, SH, C₁₋₆ alkyl;

According to an embodiment of the present disclosure, X is selected from the group consisting of the following groups which are unsubstituted or optionally substituted with one, two, or more Rₓ: OH, SH, methyl.

According to an embodiment of the present disclosure, each Rₓ is the same or different and is independently selected from H, halogen, CN, C₁₋₆ alkyl, and halo-C₁₋₆ alkyl;

According to an embodiment of the present disclosure, each Rx is the same or different and is independently selected from H, F, CN, and CF₃.

According to an embodiment of the present disclosure, X is selected from CF₃, OCF₃, SCF₃, and SCN.

According to an embodiment of the present disclosure, Y is H.

According to an embodiment of the present disclosure, R₁ is hydrogen.

According to an embodiment of the present disclosure, R₂ is selected from 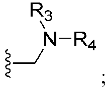 R₃ and R₄ are the same or different and are independently selected from H and C₁₋₆ alkyl; or, R₃ and R₄ together with the N to which they are attached form a 5- to 8-membered heterocyclyl, which is unsubstituted or optionally substituted with one, two, or more R₃₂;

According to an embodiment of the present disclosure, R₃ and R₄ are the same or different and are independently selected from H, methyl, ethyl, and propyl; or, R₃ and R₄ together with the N to which they are attached form pyrrolidinyl, piperazinyl, or piperidinyl, which is unsubstituted or optionally substituted with one, two, or more R₃₂;

According to an embodiment of the present disclosure, each R₃₂ is the same or different and is independently selected from H, OH, and C₁₋₆ alkyl;

According to an embodiment of the present disclosure, each R₃₂ is the same or different and is independently selected from H, OH, and methyl;

According to an embodiment of the present disclosure, R₂ is selected from

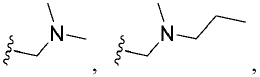

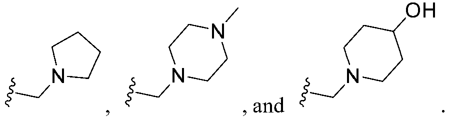

According to an embodiment of the present disclosure,

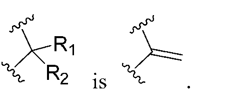

According to an embodiment of the present disclosure, the pharmaceutically acceptable salt is a pharmaceutically acceptable salt formed from the compound of formula (I) with an inorganic acid or an organic acid, including a quaternary ammonium salt formed from the compound of formula (I), wherein the inorganic acid or organic acid is selected from hydrofluoric acid, hydrochloric acid, hydrobromic acid, hydroiodic acid, sulfuric acid, phosphoric acid, nitric acid, phosphorous acid, sulfurous acid, carbonic acid, boric acid, phosphomolybdic acid, selenious acid, methanesulfonic acid, substituted methanesulfonic acid, benzenesulfonic acid, substituted benzenesulfonic acid, fumaric acid, citric acid, maleic acid, tartaric acid, oxalic acid, D-malic acid, L-malic acid, DL-malic acid, L-lactic acid, D-lactic acid, DL-lactic acid, formic acid, substituted formic acid, acetic acid, propionic acid, butyric acid, valeric acid, oleic acid, lauric acid, p-toluenesulfonic acid, 1-naphthalenesulfonic acid, 2-naphthalenesulfonic acid, phthalic acid, malonic acid, succinic acid, glycolic acid, thioglycolic acid, glycine, sarcosine, sulfonic acid, nicotinic acid, picolinic acid, isonicotinic acid, dichloroacetic acid, benzoic acid, and substituted benzoic acid.

According to an embodiment of the present disclosure, a compound of formula (I) has the following structure: 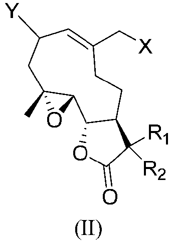 wherein X, Y, R₁, and R₂ have the definitions described in the present disclosure.

According to an embodiment of the present disclosure, a compound of formula (I) has the following structure: 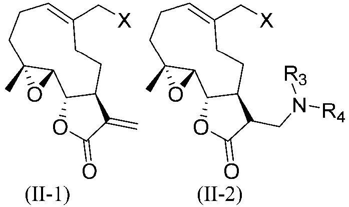 wherein X, R₃, and R₄ have the definitions described in the present disclosure.

According to an embodiment of the present disclosure, the compound of formula (I) is selected from the following structures:

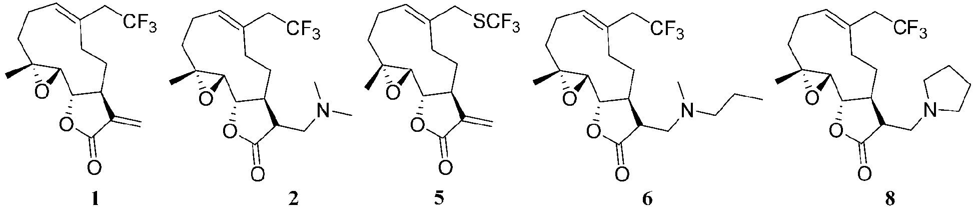

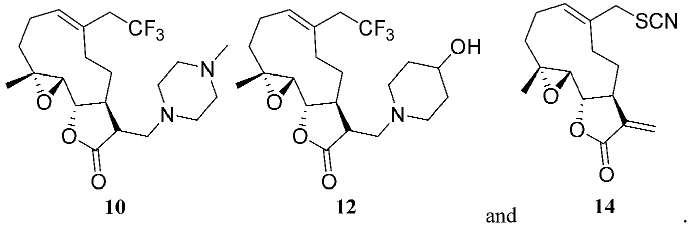

According to an embodiment of the present disclosure, a pharmaceutically acceptable salt of the compound of formula (I) is selected from the following structures:

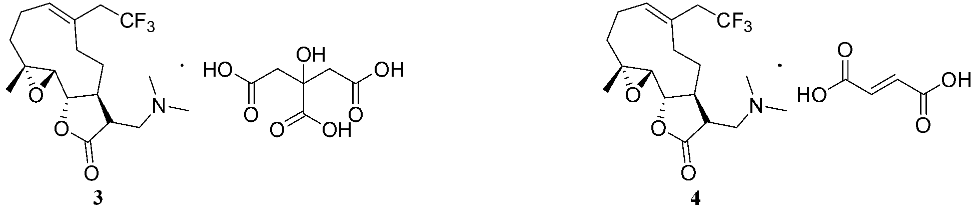

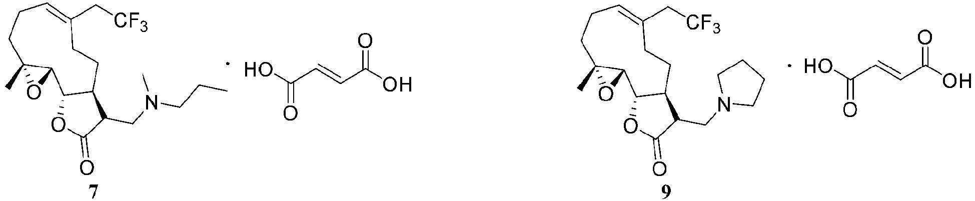

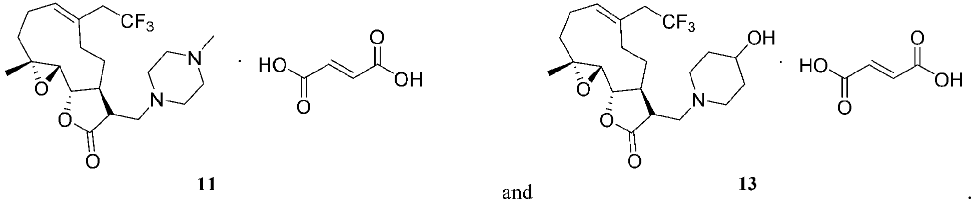

The present disclosure also provides a method for preparing a compound of formula (I), comprising the following steps: adding triphenylphosphine and a haloalkane to a solution of compound a, then adding Cu and a trifluoromethylating reagent to obtain the compound of formula (I); 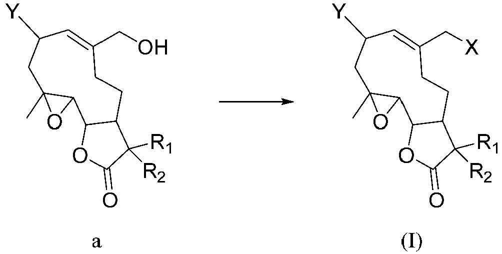 wherein X, Y, R₁, and R₂ have the definitions described in the present disclosure;

According to an embodiment of the present disclosure, the haloalkane is, for example, a dihaloalkane, preferably iodomethane, iodoethane, diiodomethane, or diiodoethane;

According to an embodiment of the present disclosure, the trifluoromethylating reagent is, for example, methyl fluorosulfonyldifluoroacetate (FSO₂CF₂CO₂Me).

According to an embodiment of the present disclosure, when 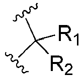 in formula (I) is 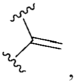 the preparation method further comprises the following step: reacting compound (I') with compound NH(R₃)(R₄) to obtain compound (I"); 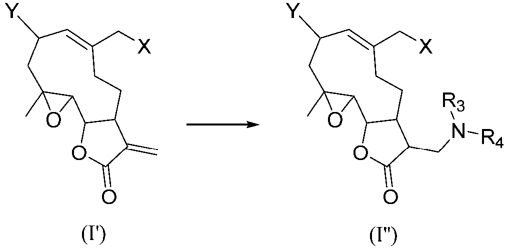 wherein X, Y, R₃, and R₄ have the definitions described in the present disclosure; when 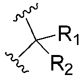 in formula (I) is 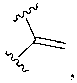 is denoted as (I'); when 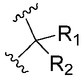 in formula (I) is 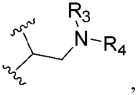 is denoted as (I").

The present disclosure also provides a method for preparing a pharmaceutically acceptable salt of the compound of formula (I), comprising the following step: reacting the compound of formula (I) with an acid to obtain the pharmaceutically acceptable salt of the compound of formula (I);

According to an embodiment of the present disclosure, the acid is selected from an inorganic acid or an organic acid, and the inorganic acid or the organic acid is hydrofluoric acid, hydrochloric acid, hydrobromic acid, hydroiodic acid, sulfuric acid, phosphoric acid, nitric acid, phosphorous acid, sulfurous acid, carbonic acid, boric acid, phosphomolybdic acid, selenious acid, methanesulfonic acid, a substituted methanesulfonic acid, benzenesulfonic acid, a substituted benzenesulfonic acid, fumaric acid, citric acid, maleic acid, tartaric acid, oxalic acid, D-malic acid, L-malic acid, DL-malic acid, L-lactic acid, D-lactic acid, DL-lactic acid, formic acid, a substituted formic acid, acetic acid, propionic acid, butyric acid, valeric acid, oleic acid, lauric acid, p-toluenesulfonic acid, 1-naphthalenesulfonic acid, 2-naphthalenesulfonic acid, phthalic acid, malonic acid, succinic acid, glycolic acid, thioglycolic acid, glycine, sarcosine, sulfonic acid, nicotinic acid, picolinic acid, isonicotinic acid, dichloroacetic acid, benzoic acid, and a substituted benzoic acid.

The present disclosure also provides a pharmaceutical composition, comprising a compound represented by formula (I), a deuterated form thereof, a tautomer thereof, a stereoisomer thereof, a hydrate thereof, a solvate thereof, a pharmaceutically acceptable salt thereof, or a prodrug thereof.

According to an embodiment of the present disclosure, the pharmaceutical composition further comprises a pharmaceutically acceptable carrier.

According to an embodiment of the present disclosure, the pharmaceutical composition further comprises another drug for treating cancer.

The present disclosure also provides a use of a compound represented by formula (I), a deuterated form thereof, a tautomer thereof, a stereoisomer thereof, a hydrate thereof, a solvate thereof, a pharmaceutically acceptable salt thereof, or a prodrug thereof in the preparation of a drug.

According to an embodiment of the present disclosure, the drug is a drug for treating cancer.

According to an embodiment of the present disclosure, the cancer includes: leukemia, breast cancer, nasopharyngeal carcinoma, colorectal cancer, lung cancer, liver cancer, esophageal cancer, gastric cancer, intestinal cancer, kidney cancer, oral cancer, colorectal cancer, glioma, melanoma, bladder cancer, ovarian cancer, thyroid cancer, blood cancer, bone cancer, brain cancer, central nervous system cancer, cervical cancer, colon cancer, endometrial cancer, esophageal cancer, gallbladder cancer, gastrointestinal cancer, cancer of the external genitalia, genitourinary tract cancer, head cancer, laryngeal cancer, muscle tissue cancer, neck cancer, oral or nasal mucosal cancer, pancreatic cancer, prostate cancer, skin cancer, spleen cancer, small intestine cancer, testicular cancer.

The present disclosure also provides a compound represented by formula (I), a deuterated form thereof, a tautomer thereof, a stereoisomer thereof, a hydrate thereof, a solvate thereof, a pharmaceutically acceptable salt thereof, a prodrug thereof, or the pharmaceutical composition for preventing and/or treating cancer.

The present disclosure also provides a method for preventing and/or treating cancer, comprising administering to a patient a therapeutically effective amount of at least one of a compound represented by formula (I), a deuterated form thereof, a tautomer thereof, a stereoisomer thereof, a hydrate thereof, a solvate thereof, a pharmaceutically acceptable salt thereof, a prodrug thereof, or the pharmaceutical composition.

According to an embodiment of the present disclosure, the cancer includes: leukemia, breast cancer, nasopharyngeal carcinoma, colorectal cancer, lung cancer, liver cancer, esophageal cancer, gastric cancer, intestinal cancer, kidney cancer, oral cancer, colorectal cancer, glioma, melanoma, bladder cancer, ovarian cancer, thyroid cancer, blood cancer, bone cancer, brain cancer, central nervous system cancer, cervical cancer, colon cancer, endometrial cancer, esophageal cancer, gallbladder cancer, gastrointestinal cancer, cancer of the external genitalia, genitourinary tract cancer, head cancer, laryngeal cancer, muscle tissue cancer, neck cancer, oral or nasal mucosal cancer, pancreatic cancer, prostate cancer, skin cancer, spleen cancer, small intestine cancer, testicular cancer.

According to an embodiment of the present disclosure, the carrier is one or more solid, semi-solid, liquid formulations, and pharmaceutical excipients. The drug of the present disclosure can be administered in two forms: injection and oral administration. The injection can be intravenous injection or intramuscular injection, and the oral dosage form can be a tablet or a capsule.

In preparing the drug described in the present disclosure, the active compound is combined or formulated with an appropriate pharmaceutically acceptable carrier, diluent, or excipient, and can be formulated into preparations in solid, semi-solid, liquid, or gaseous forms, such as tablets, capsules, powders, granules, ointments, solutions, suppositories, injections, inhalations, gels, microspheres, and aerosols. The administration routes include oral, intraperitoneal, transdermal, subcutaneous, intravenous or intramuscular injection, inhalation, topical, intralesional, infusion; liposome-mediated delivery; topical, intrathecal, gingival pocket, rectal, endobronchial, nasal, transmucosal, enteral, ocular or otic delivery, or any other method known in the art, all of which are capable of achieving therapeutic effects on tumors;
The therapeutically effective amount or dose described in the present disclosure will vary depending on several factors, including the selected administration route, the formulation of the composition, the patient's response, the severity of the condition, the subject's body weight, and the prescribing physician's judgment, for example, 1-200 mg/kg, 40-150 mg/kg, such as 50 mg/kg. The dose can be increased or decreased over time, as required by individual patients. In some cases, patients are initially given a low dose, which is then increased to an effective dose tolerable to the patient. Furthermore, patients can be given multiple doses over a defined period, particularly at time increments (such as daily, weekly, biweekly, monthly, quarterly, biennially, or the like).

### Advantageous Effects

The present disclosure provides a fluorinated parthenolide derivative compound shown in formula (I), a deuterated form, tautomer, stereoisomer, hydrate, solvate, pharmaceutically acceptable salt, or prodrug thereof, which has good tumor inhibitory effects and can be used for preventing and/or treating cancer. The preparation method provided by the present disclosure is simple to operate and can efficiently and rapidly construct the fluorinated parthenolide molecule.

### BRIEF DESCRIPTION OF THE DRAWINGS

FIG. 1 Release profile of compound 4 in a buffer solution.
FIG. 2 Plasma concentration-time curves of compound 4 after a single oral gavage and intravenous injection administration.

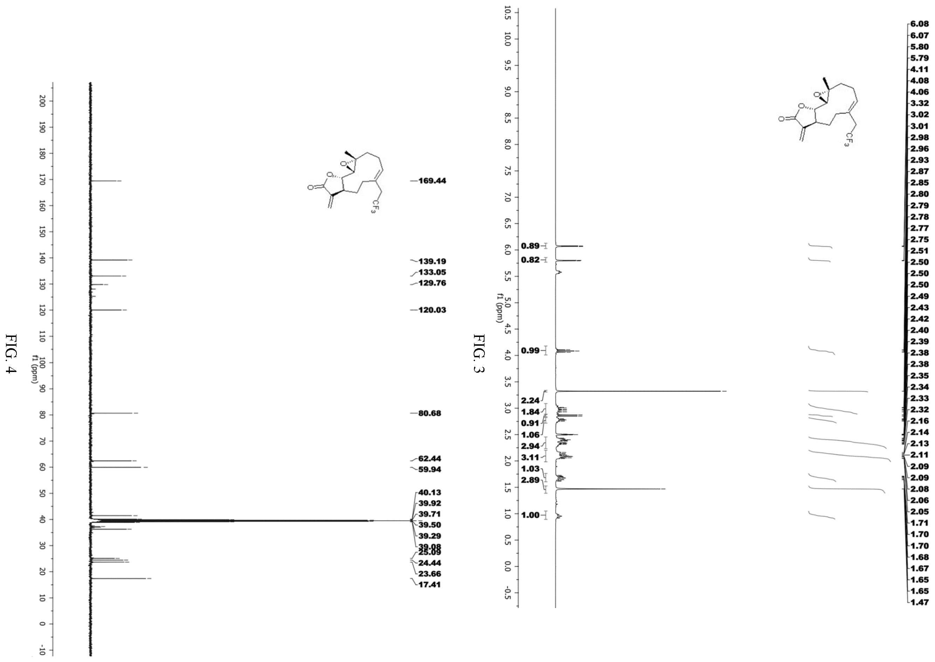

FIG. 3 is the ¹H NMR spectrum of compound 1;
FIG. 4 is the ¹³C NMR spectrum of compound 1;

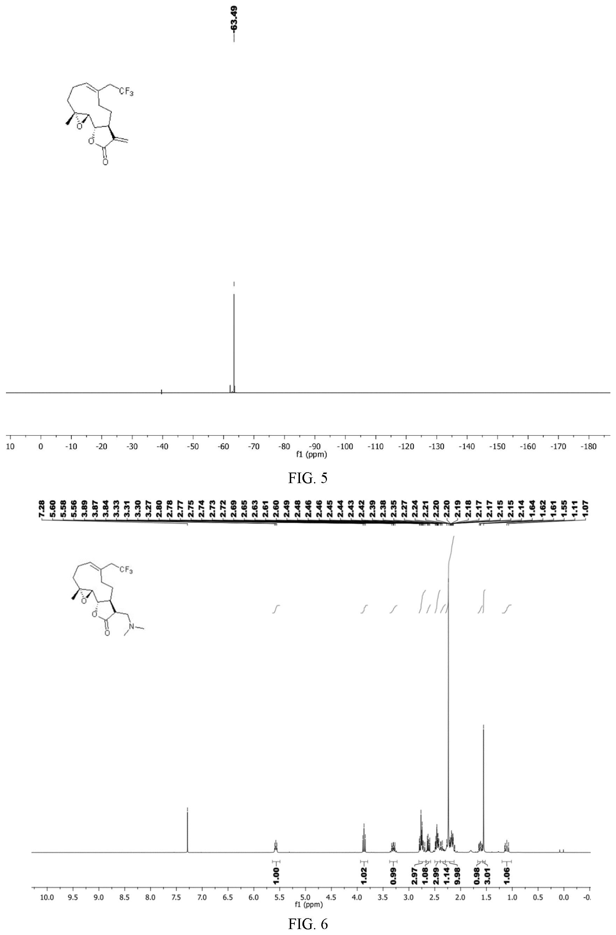

FIG. 5 is the ¹⁹F NMR spectrum of compound 1;
FIG. 6 is the ¹H NMR spectrum of compound 2;

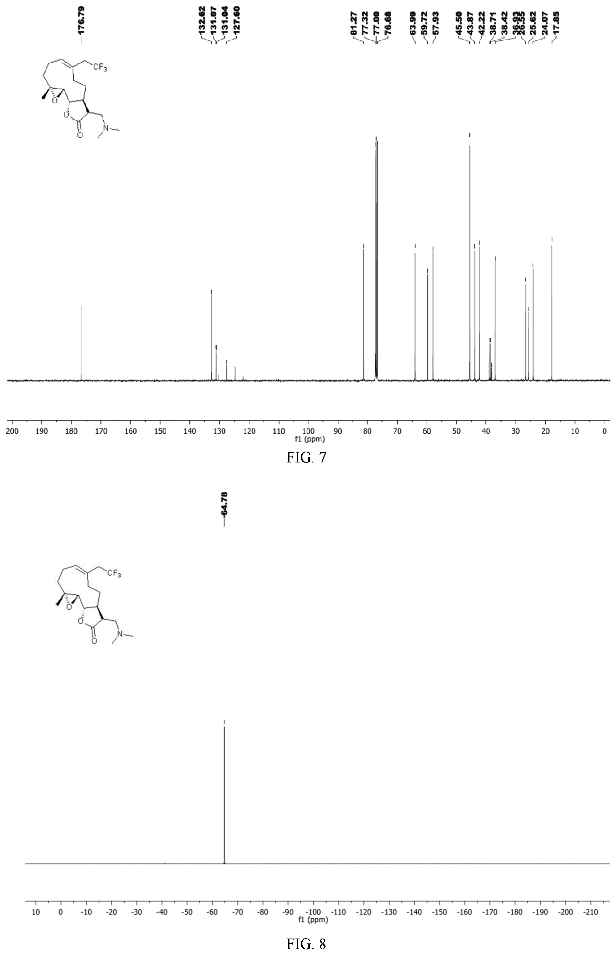

FIG. 7 is the ¹³C NMR spectrum of compound 2;
FIG. 8 is the ¹⁹F NMR spectrum of compound 2;

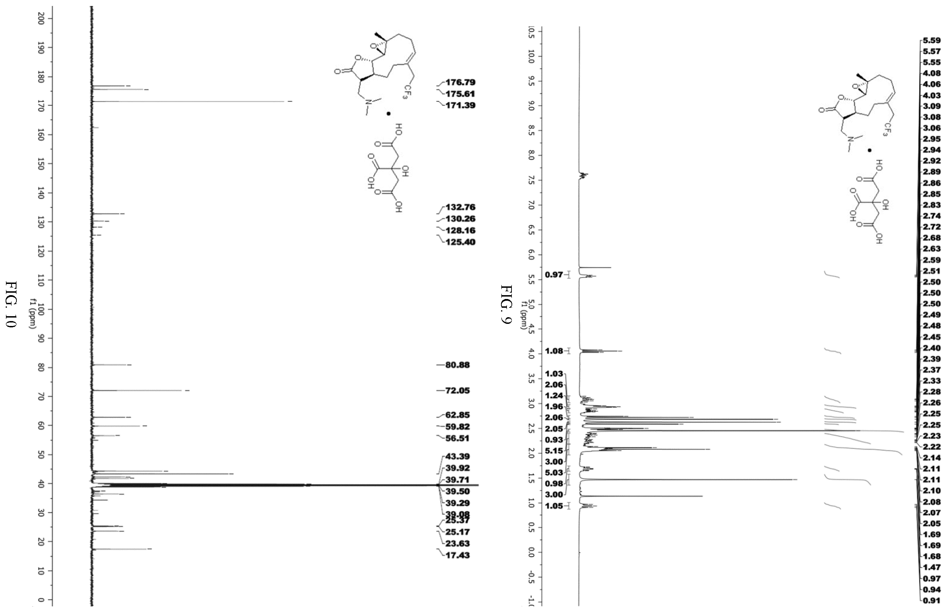

FIG. 9 is the ¹H NMR spectrum of compound 3;
FIG. 10 is the ¹³C NMR spectrum of compound 3;

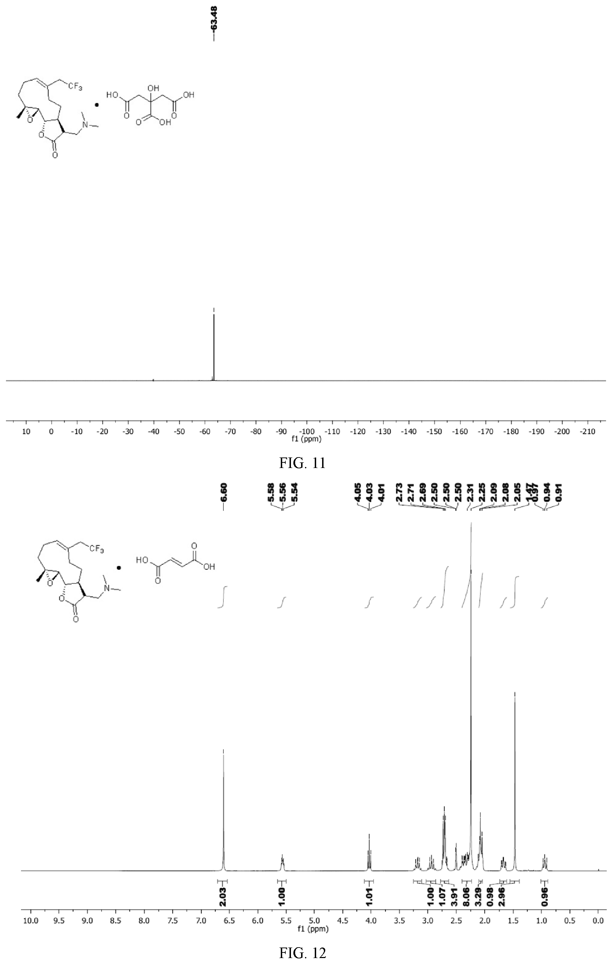

FIG. 11 is the ¹⁹F NMR spectrum of compound 3;
FIG. 12 is the ¹H NMR spectrum of compound 4;

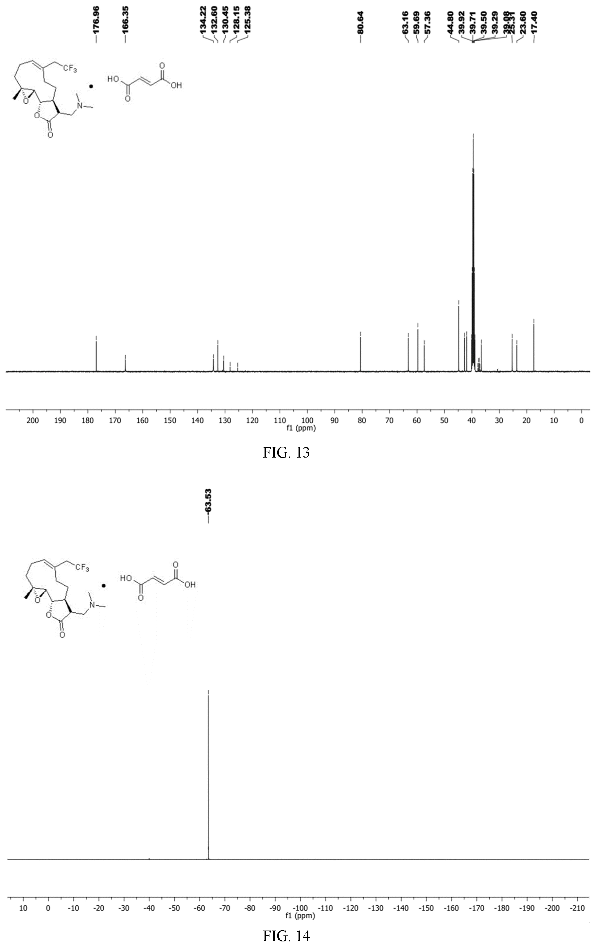

FIG. 13 is the ¹³C NMR spectrum of compound 4;
FIG. 14 is the ¹⁹F NMR spectrum of compound 4;

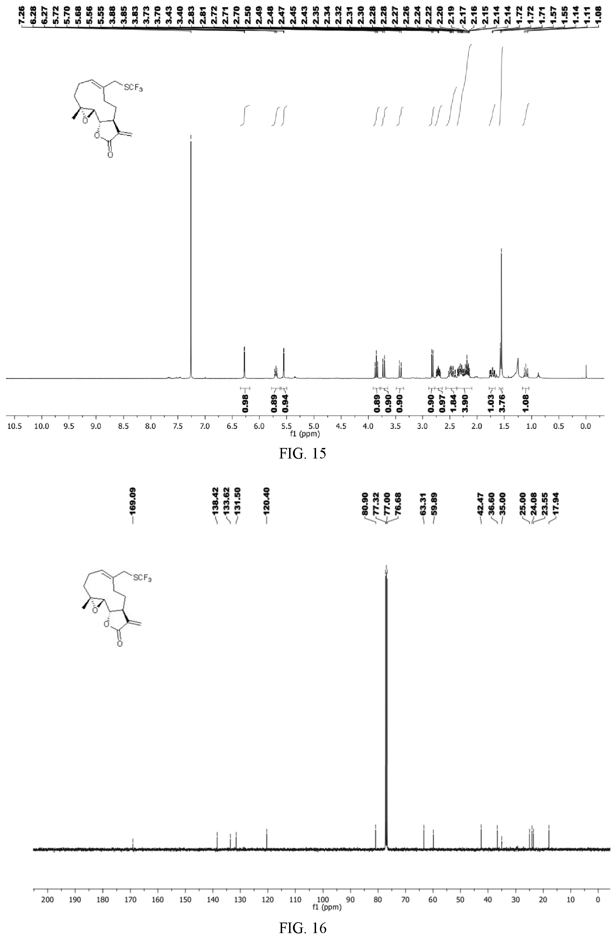

FIG. 15 is the ¹H NMR spectrum of compound 5;
FIG. 16 is the ¹³C NMR spectrum of compound 5;

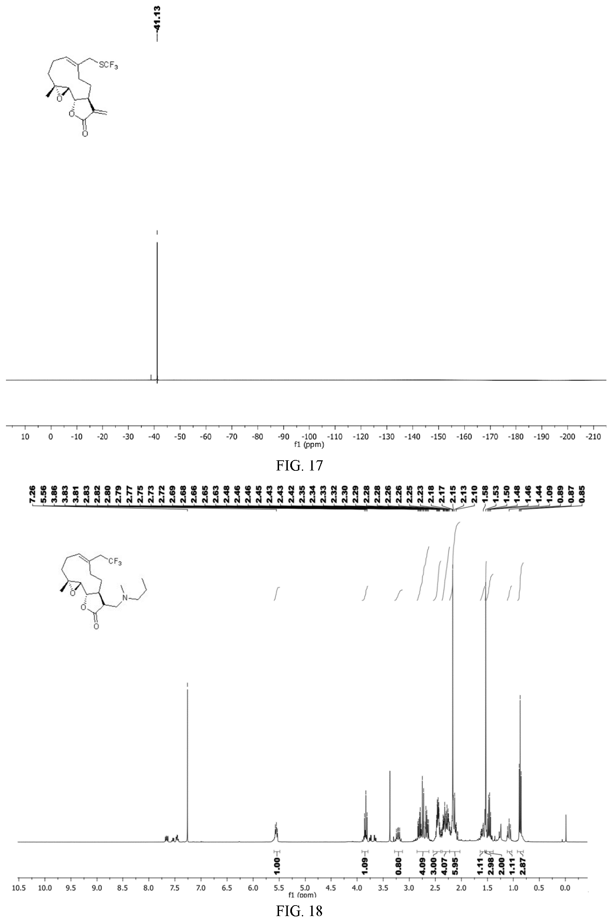

FIG. 17 is the ¹⁹F NMR spectrum of compound 5;
FIG. 18 is the ¹H NMR spectrum of compound 6;

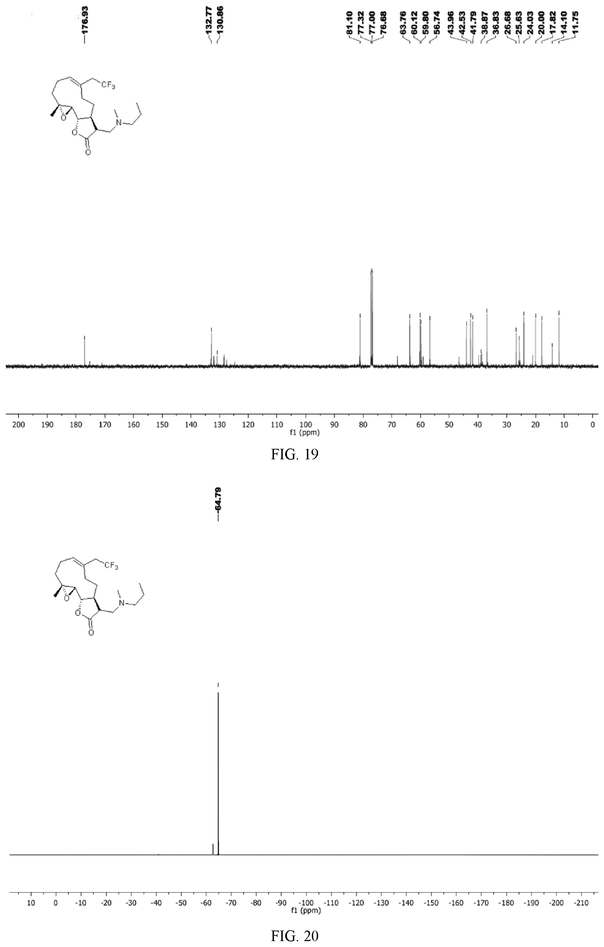

FIG. 19 is the ¹³C NMR spectrum of compound 6;
FIG. 20 is the ¹⁹F NMR spectrum of compound 6;

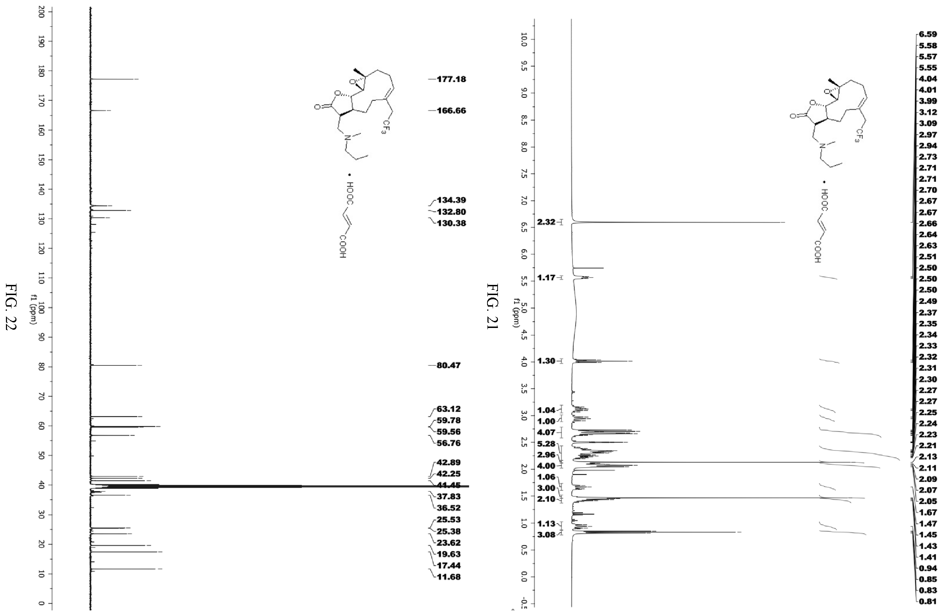

FIG. 21 is the ¹H NMR spectrum of compound 7;
FIG. 22 is the ¹³C NMR spectrum of compound 7;

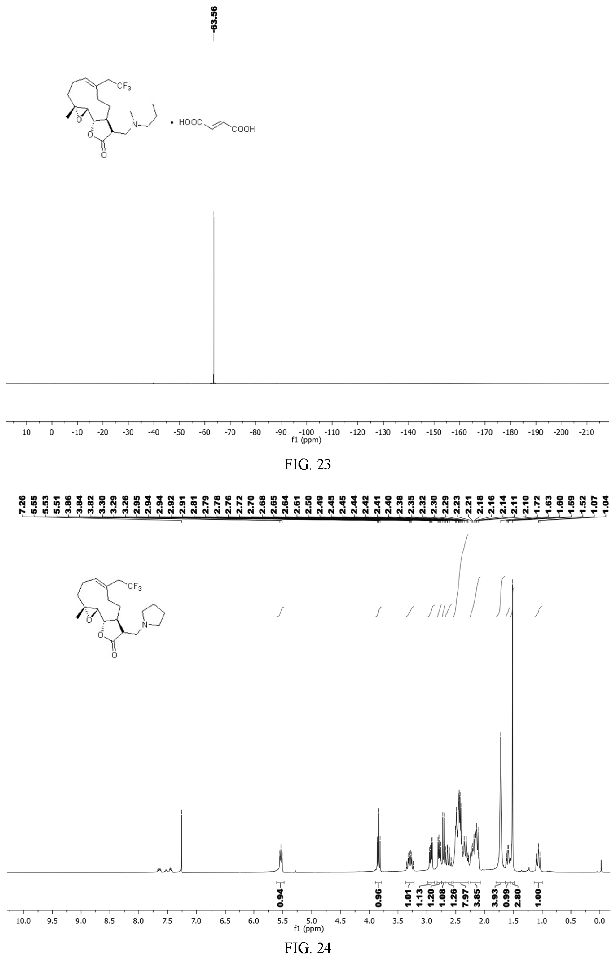

FIG. 23 is the ¹⁹F NMR spectrum of compound 7;
FIG. 24 is the ¹H NMR spectrum of compound 8;

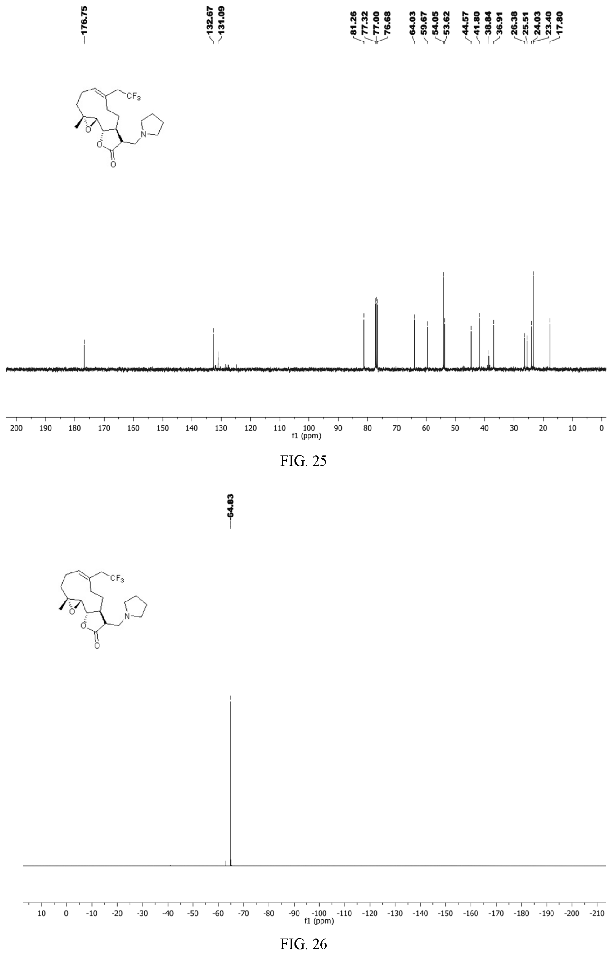

FIG. 25 is the ¹³C NMR spectrum of compound 8;
FIG. 26 is the ¹⁹F NMR spectrum of compound 8;

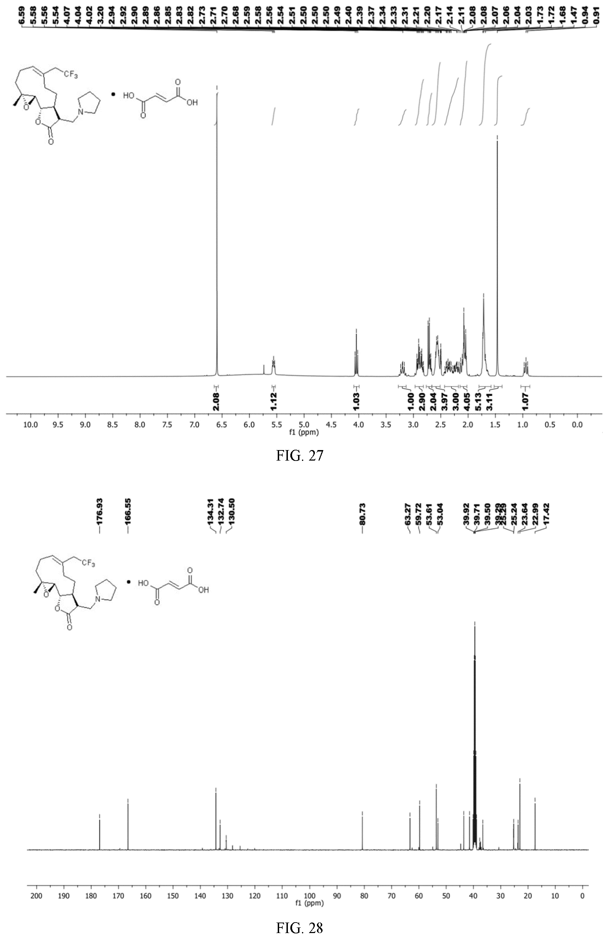

FIG. 27 is the ¹H NMR spectrum of compound 9;
FIG. 28 is the ¹³C NMR spectrum of compound 9;

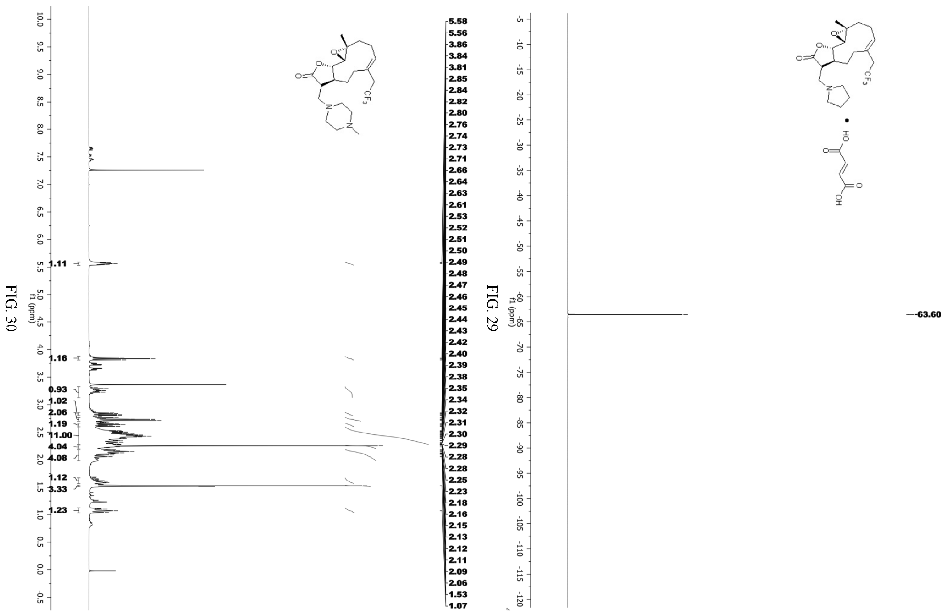

FIG. 29 is the ¹⁹F NMR spectrum of compound 9;
FIG. 30 is the ¹H NMR spectrum of compound 10;

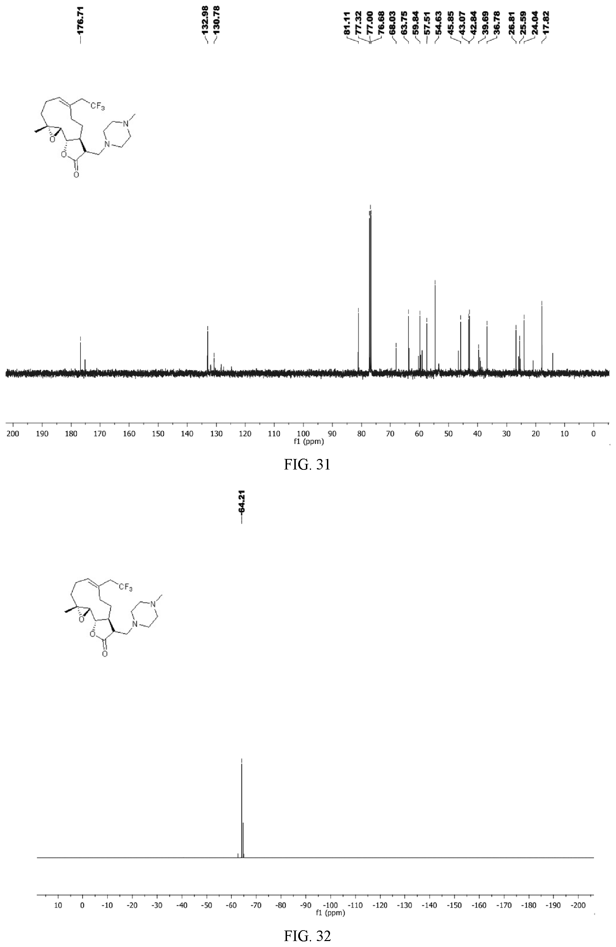

FIG. 31 is the ¹³C NMR spectrum of compound 10;
FIG. 32 is the ¹⁹F NMR spectrum of compound 10;

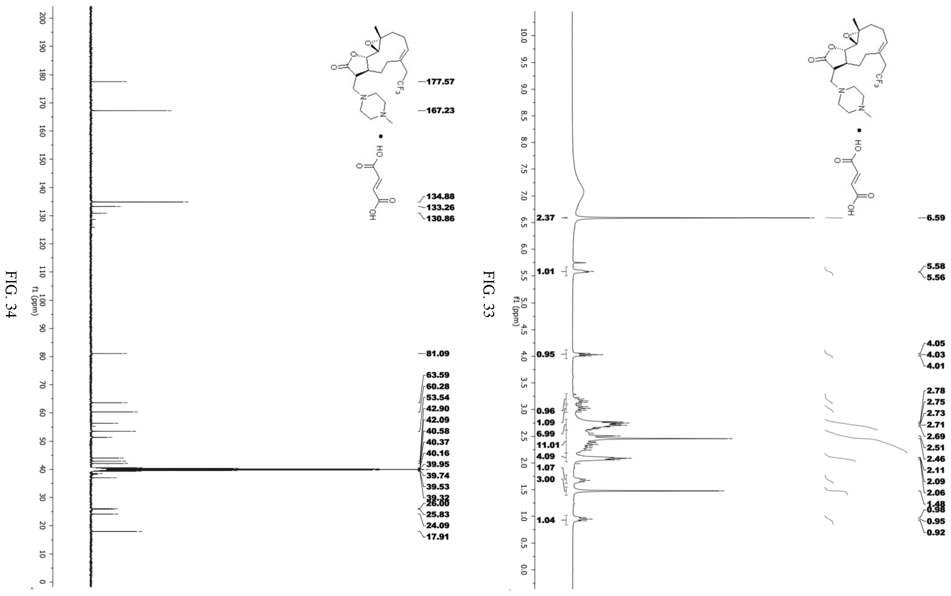

FIG. 33 is the ¹H NMR spectrum of compound 11;
FIG. 34 is the ¹³C NMR spectrum of compound 11;

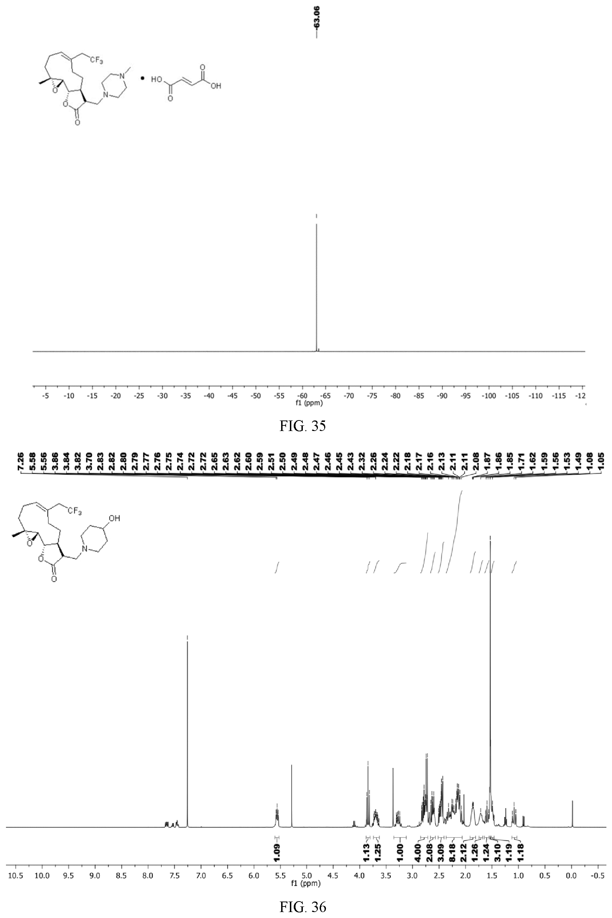

FIG. 35 is the ¹⁹F NMR spectrum of compound 11;
FIG. 36 is the ¹H NMR spectrum of compound 12;

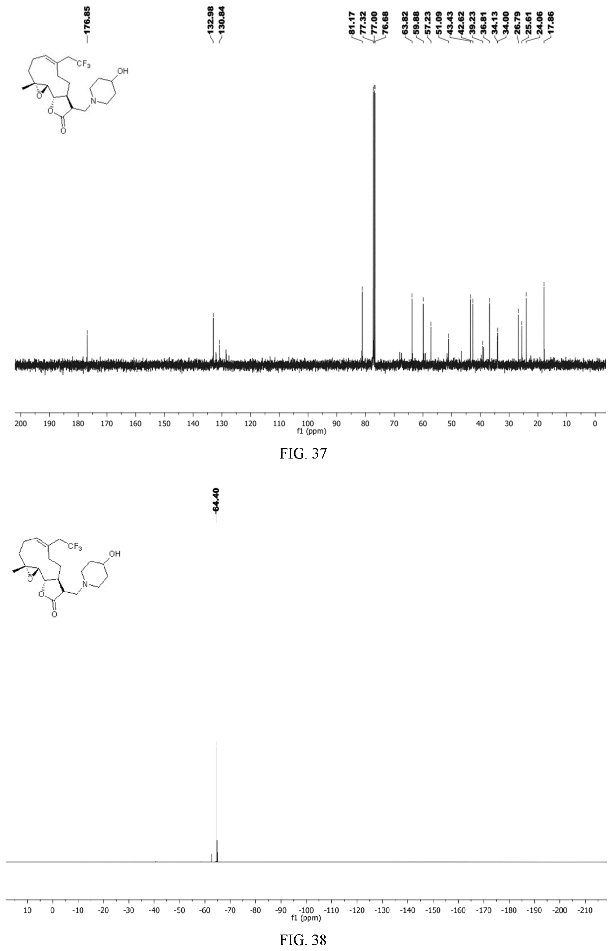

FIG. 37 is the ¹³C NMR spectrum of compound 12;
FIG. 38 is the¹⁹F NMR spectrum of compound 12;

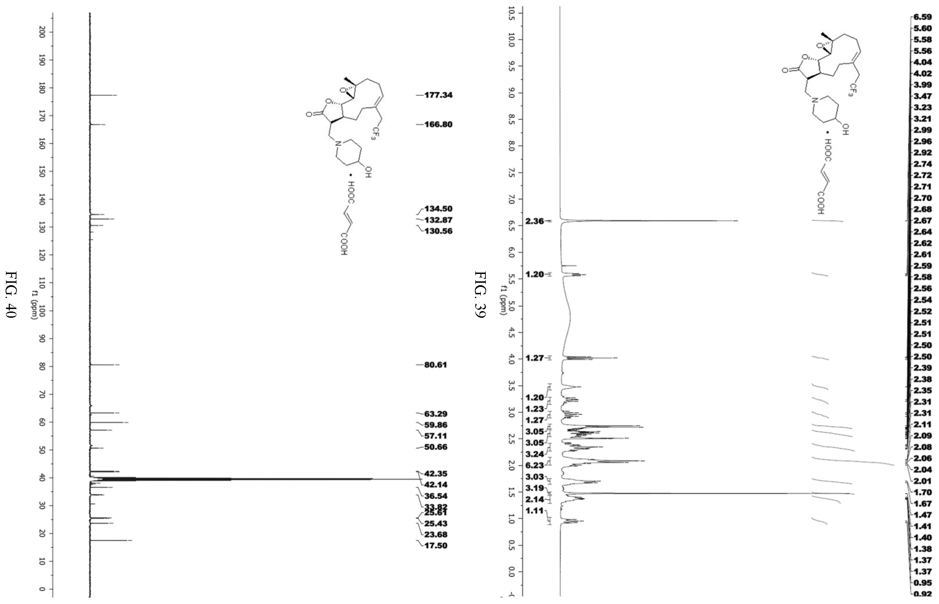

FIG. 39 is the ¹H NMR spectrum of compound 13;
FIG. 40 is the ¹³C NMR spectrum of compound 13;

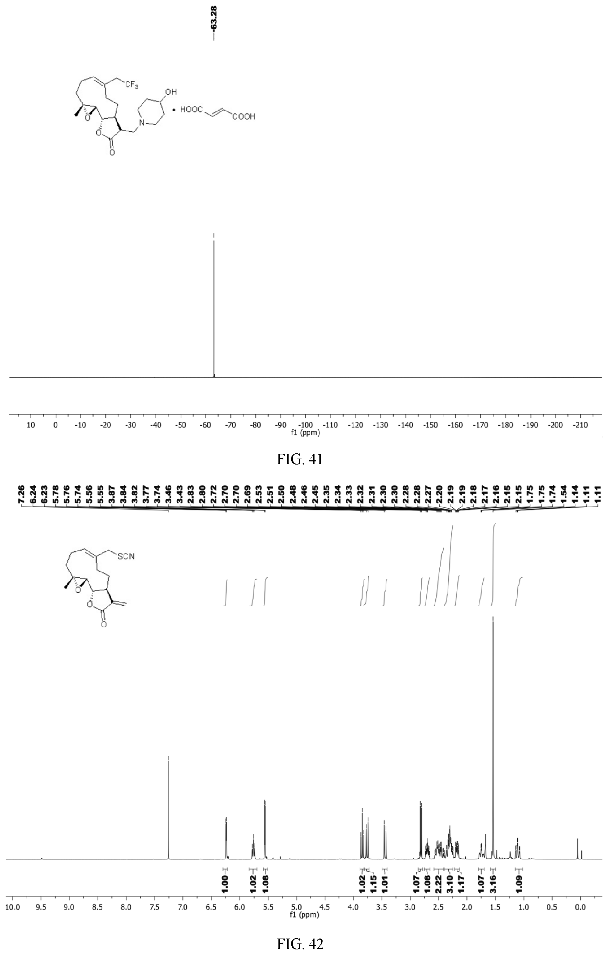

FIG. 41 is the ¹⁹F NMR spectrum of compound 13;
FIG. 42 is the ¹H NMR spectrum of compound **14;**

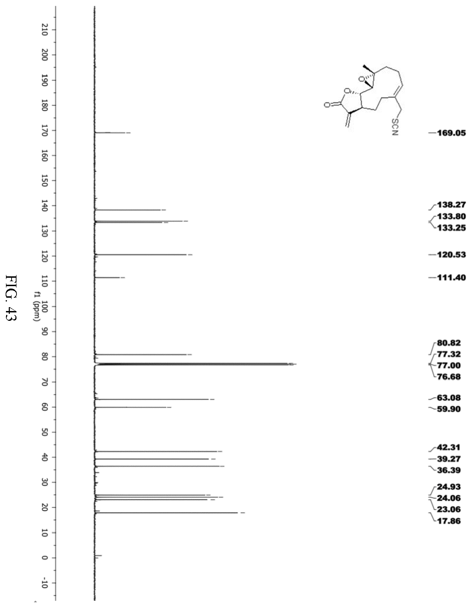

FIG. 43 is the ¹³C NMR spectrum of compound **14.**

### Definition and Explanation of Terms

Unless otherwise stated, the definitions of groups and terms described in the specification and claims of the present application, including definitions thereof as examples, exemplary definitions, preferred definitions, definitions documented in tables, definitions of specific compounds in the examples, and the like, may be arbitrarily combined and incorporated with each other. The definitions of groups and the structures of the compounds in such combinations and incorporations should be understood to fall within the scope of the specification and/or claims of the present application.

It should be understood that, as used herein in describing one, two, or more, "more" should mean greater than 2, for example, an integer greater than or equal to 3, such as 3, 4, 5, 6, 7, 8, 9, or 10.

The term "C₁₋₁₀ alkyl" denotes a straight or branched chain saturated hydrocarbon group having 1, 2, 3, 4, 5, 6, 7, 8, 9, or 10 carbon atoms. The C₁₋₁₀ alkyl includes C₁₋₃ alkyl, C₁₋₆ alkyl, C₃₋₆ alkyl, and the like. "C₁₋₁₀ alkyl" denotes a straight and branched chain alkyl group having 1, 2, 3, 4, 5, 6, 7, 8, 9, or 10 carbon atoms; "C₁₋₈ alkyl" denotes a straight and branched chain alkyl group having 1, 2, 3, 4, 5, 6, 7, or 8 carbon atoms; "C₁₋₆ alkyl" denotes a straight and branched chain alkyl group having 1, 2, 3, 4, 5, or 6 carbon atoms. The alkyl group is, for example, methyl, ethyl, propyl, butyl, pentyl, hexyl, isopropyl, isobutyl, sec-butyl, tert-butyl, isopentyl, 2-methylbutyl, 1-methylbutyl, 1-ethylpropyl, 1,2-dimethylpropyl, neopentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl, or 1,2-dimethylbutyl, or an isomer thereof.

The term "C₃₋₁₀ cycloalkyl" denotes a saturated monovalent monocyclic, bicyclic (such as fused, bridged, or spiro) hydrocarbon ring or tricyclic alkane having 3, 4, 5, 6, 7, 8, 9, or 10 carbon atoms. The C₃₋₁₀ cycloalkyl includes C₃₋₈ cycloalkyl, C₃₋₅ cycloalkyl, C₆₋₈ cycloalkyl, C₃₋₄ cycloalkyl, C₅₋₆ cycloalkyl, C₆ cycloalkyl, and the like. The C₃₋₁₀ cycloalkyl can be a monocyclic hydrocarbon group, such as cyclopropyl, cyclobutyl, cyclopentyl, cyclohexyl, cycloheptyl, cyclooctyl, cyclononyl, or cyclodecyl; or a bicyclic hydrocarbon group such as bornyl, indanyl, hexahydroindanyl, tetrahydronaphthyl, decahydronaphthyl, bicyclo[2.1.1]hexyl, bicyclo[2.2.1]heptyl, bicyclo[2.2.1]heptenyl, 6,6-dimethylbicyclo[3.1.1]heptyl, 2,6,6-trimethylbicyclo[3.1.1]heptyl, bicyclo[2.2.2]octyl, 2,7-diazaspiro[3.5]nonanyl, 2,6-diazaspiro[3.4]octanyl; or a tricyclic hydrocarbon group such as adamantyl.

The term "3-10 membered heterocyclyl" means a saturated or unsaturated non-aromatic ring or ring system containing at least one heteroatom selected from O, S, and N. The heterocyclyl can be attached to the rest of the molecule via any one of the carbon atoms or a nitrogen atom, if present. The heterocyclyl can include fused or bridged rings as well as spiro rings. In particular, the heterocyclyl can include, but is not limited to: 4-membered rings, such as azetidinyl, oxetanyl; 5-membered rings, such as tetrahydrofuranyl, dioxolyl, pyrrolidinyl, imidazolidinyl, pyrazolidinyl, pyrrolinyl; or 6-membered rings, such as tetrahydropyranyl, piperidinyl, morpholinyl, dithianyl, thiomorpholinyl, piperazinyl, or trithianyl; or 7-membered rings, such as diazepanyl. Optionally, the heterocyclyl group can be benzofused. The heterocyclyl group can be bicyclic, for example, but not limited to, a 5,5-membered ring such as a hexahydrocyclopenta[c]pyrrol-2(1H)-yl ring, or a 5,6-membered bicyclic ring such as a hexahydropyrrolo[1,2-a]pyrazin-2(1H)-yl ring. The heterocyclyl group can be partially unsaturated, i.e., it can contain one or more double bonds, for example, but not limited to, a dihydrofuranyl group, a dihydropyranyl group, a 2,5-dihydro-1H-pyrrolyl group, a 4H-[1,3,4]thiadiazinyl group, a 1,2,3,5-tetrahydrooxazolyl group, or a 4H-[1,4]thiazinyl group; or, it can be benzofused, for example, but not limited to, a dihydroisoquinolinyl group.

The term "C₆₋₁₄ aryl" is understood to preferably mean a monocyclic, bicyclic, or tricyclic hydrocarbon ring that is aromatic or partially aromatic and has 6, 7, 8, 9, 10, 11, 12, 13, or 14 carbon atoms ("C₆₋₁₄ aryl"), particularly a ring having 6 carbon atoms ("C₆ aryl"), for example, a phenyl group; or a biphenyl group, or a ring having 9 carbon atoms ("C₉ aryl"), for example, an indanyl group or an indenyl group, or a ring having 10 carbon atoms ("C₁₀ aryl"), for example, a tetrahydronaphthyl group, a dihydronaphthyl group, or a naphthyl group, or a ring having 13 carbon atoms ("C₁₃ aryl"), for example, a fluorenyl group, or a ring having 14 carbon atoms ("C₁₄ aryl"), for example, an anthryl group. When the C₆₋₂₀ aryl group is substituted, it can be monosubstituted or polysubstituted. Furthermore, there is no limitation on its substitution site, for example, it can be ortho-, para-, or meta-substituted.

The term "5-14 membered heteroaryl" means a monovalent or polyvalent monocyclic, bicyclic, or tricyclic aromatic ring system having 5, 6, 7, 8, 9, 10, 11, 12, 13, or 14 ring atoms, and whose ring atoms contain 1 to 5 heteroatoms independently selected from N, O, and S, wherein the bicyclic and tricyclic aromatic ring systems can be fused rings, spiro rings, or bridged rings. The number of heteroatoms in the 5-14 membered heteroaryl group is 1 to 5, preferably 1 to 3. Additionally, in each case, the 5-14 membered heteroaryl group can be benzofused. The 5-14 membered heteroaryl group includes a 5-8 membered heteroaryl group, a 5-9 membered heteroaryl group, a 5-10 membered heteroaryl group, a 5-6 membered heteroaryl group, an 8-10 membered heteroaryl group, a 6-membered heteroaryl group, and the like. Examples of heteroaryl include, but are not limited to: 5-membered rings, such as oxazolyl, pyrazolyl, thienyl, thiazolyl, triazolyl, imidazolyl, and the like; 6-membered rings, such as pyridyl, pyrimidinyl, pyrazinyl, pyridazinyl, and the like. The heterocyclyl can be bicyclic, including, but not limited to: 5,5-membered rings, such as tetrahydrocyclopentapyrazole; 5,6-membered rings, such as tetrahydroindole, tetrahydropyrazolopyridine, tetrahydroimidazopyridine, tetrahydrobenzisoxazole, tetrahydrobenzoxazole, tetrahydrobenzothiazole, tetrahydrobenzisothiazole, dihydrofuropyrazole, tetrahydrobenzofuran, dihydrobenzofuran, tetrahydrobenzothiophene; 6,6-membered rings, such as tetrahydroquinoline; 5,7-membered rings, such as tetrahydrocycloheptathiazole, tetrahydrocycloheptafuran. The heterocyclyl can be tricyclic, including, but not limited to: 6,7-dihydrospiro[cyclopropane-1,5-pyrrolo[1,2-c]imidazole]. When the 5-14 membered heteroaryl is substituted, it can be monosubstituted or polysubstituted. Furthermore, there is no limitation on the substitution site; for example, a hydrogen atom bonded to a carbon atom on the heteroaryl ring can be substituted, or a hydrogen atom bonded to a heteroatom on the heteroaryl ring can be substituted.

The term "spiro ring" refers to a ring system in which two rings share one ring-forming atom.

The term "fused ring" refers to a ring system in which two rings share two ring-forming atoms.

The term "bridged ring" refers to a ring system in which two rings share three or more ring-forming atoms.

The term "halogen" denotes fluorine, chlorine, bromine, and iodine.

"Halo" refers to substitution by one or more halogens.

The term "halo C₁₋₁₀ alkyl" refers to an alkyl group as defined above, which is substituted by one or more halogens as defined above. The haloalkyl group includes, but is not limited to, fluoromethyl, difluoromethyl, trifluoromethyl, trichloromethyl, 2,2,2-trifluoroethyl, 2,2-difluoroethyl, 1-fluoromethyl-2-fluoroethyl, 3-bromo-2-fluoropropyl, 1-bromomethyl-2-bromoethyl, and the like.

The term "C₁₋₁₀ alkoxy" refers to a group -O-R^{X}, wherein R^{X} is an alkyl group as defined above.

### DETAILED DESCRIPTION

The technical solutions of the present disclosure are further described in detail below with reference to specific examples. It will be appreciated that the following examples are merely exemplary illustrations and explanations of the present disclosure and should not be construed as limiting the claimed scope of the present disclosure. All techniques implemented on the basis of the content described above of the present disclosure fall within the claimed scope of the present disclosure.

Unless otherwise stated, the starting materials and reagents used in the following examples are all commercially available products or can be prepared by using known methods.

### Synthesis of Compounds:

### Example 1 Preparation of Compound 1:

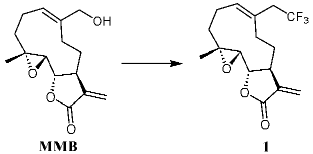

MMB (132.0 mg, 0.5 mmol), Ph₃P (157.4 mg, 0.6 mmol), and ICH₂CH₂I (169.1 mg, 0.6 mmol) were added to a Schlenk tube; nitrogen was introduced; and then DMF (5 mL) was added. After stirring at room temperature until complete dissolution, Cu (31.8 mg, 0.5 mmol) and FSO₂CF₂CO₂Me (480.2 mg, 2.5 mmol) were added sequentially. The reaction mixture was stirred at 80 °C for 2 hours. The reaction mixture was cooled to room temperature and filtered through a fritted funnel. The filtrate was concentrated, and the resulting crude product was purified on a silica gel column (petroleum ether:ethyl acetate = 8:1) to afford compound 1 as a white solid. Yield: 50 mg, 32%. Melting point: 230-231 °C. ¹H NMR (400 MHz, DMSO-*d*₆): *δ* = 6.07 (d, *J **=*** 3.5 Hz, 1H), 5.80 (d, *J =* 3.2 Hz, 1H), 5.58 (t, *J* = 8 Hz, 1H), 4.08 (t, *J =* 9.3 Hz, 1H), 3.09-2.89 (m, 2H), 2.86 (d, *J =* 9.5 Hz, 1H), 2.82-2.72 (m, 1H), 2.45-2.23 (m, 3H), 2.20-1.98 (m, 3H), 1.76-1.60 (m, 1H), 1.47 (s, 3H), 0.96 (m, 1H).¹³C NMR (100 MHz, DMSO-*d*₆): *δ* = 169.4, 139.2, 133.1, 129.8, 120.0, 80.7, 62.4, 59.9, 41.5, 37.3(q, *J* = 37.15 Hz), 36.3, 25.1, 24.4, 23.7, 17.4. ¹⁹F NMR (376 MHz, DMSO-*d*₆) *δ* = -63.49 (s, 3F). HRMS (ESI): m/z [M + H]⁺ calcd for: C₁₆H₂₀F₃O₃: 317.1359; found: 317.1354.

### Example 2 Preparation of Compound 2:

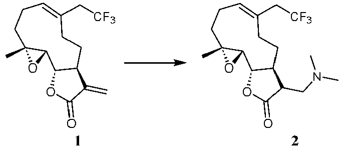

Compound 1 (316.1 mg, 1 mmol), Me₂NH·HCl (652.4 mg, 8 mmol), and potassium carbonate (2073.2 mg, 15 mmol) were added to a round-bottom flask, followed by CH₂Cl₂ (10 mL). The reaction was stirred at room temperature for 2 h. The reaction mixture was filtered through a fritted funnel and then diluted with 50 mL of dichloromethane. The organic phase was washed with brine and dried over anhydrous Na₂SO₄. The organic phase was concentrated, and the resulting crude product was purified on a silica gel column (petroleum ether:ethyl acetate = 4:1) to afford a white solid compound. Yield: 325.0 mg, 90%. Melting point: 102-103 °C. ¹H NMR (400 MHz, CDCl₃) *δ* = 5.54 (t*, J =* 8.0 Hz, 1H), 3.83 (t, *J =* 9.6 Hz, 1H), 3.31-3.19 (m, 1H), 2.79-2.64 (m, 3H), 2.64-2.51 (m, 2H), 2.51-2.27 (m, 4H), 2.24-2.09 (m, 10H), 1.63-1.54 (m, 1H), 1.52 (s, 3H), 1.07 (t, *J =* 12.5 Hz, 1H). ¹³C NMR (100 MHz, CDCl₃): δ = 176.8, 132.6, 131.0 (q, *J* = 3Hz), 127.6, 81.3, 64.0, 59.7, 57.9, 45.5, 43.9, 42.2, 38.6 (q, *J* = 28Hz), 36.9, 26.6, 25.6, 24.1, 17.9. ¹⁹F NMR (376 MHz, CDCl₃) δ = -64.78 (s, 3F). HRMS (ESI): m/z [M + H]⁺ calcd for: C₁₈H₂₇F₃NO₃: 362.1938; found: 362.1934.

### Example 3 Preparation of Compound 3:

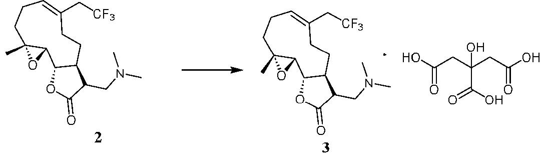

Compound 2 (361.2 mg, 1 mmol) and citric acid (192.13 mg, 1 mmol) were added to a round-bottom flask, followed by acetone (10 mL). The reaction was stirred at room temperature for 0.5 h. The reaction mixture was concentrated to afford a white solid. Yield: 525.5 mg, 95%. Melting point: 69-70 °C. 1H NMR (400 MHz, DMSO-^{d}6) _{δ} = 5.57 (t, *J =* 8.0 Hz, 1H), 4.06 (t, *J =* 9.5 Hz, 1H), 3.15-3.01 (m, 1H), 2.98-2.91 (m, 2H), 2.86 (dd, *J =* 14.3, 9.0 Hz, 1H), 2.75-2.67 (m, 4H), 2.61 (d, *J* = 15.3 Hz, 3H), 2.47 (m, 5H), 2.40-2.19 (m, 3H), 2.18-1.99 (m, 5H), 1.74-1.64 (m, 1H), 1.47 (s, 3H), 1.01-0.87 (m, 1H). ¹³C NMR (100 MHz, DMSO-d₆): *δ* = 176.8, 175.6, 171.4, 132.8, 130.3, 126.8 (d, *J =* 276 Hz), 80.9, 72.1, 62.9, 59.8, 56.5, 44.4, 43.4, 42.4, 41.8, 37.5, 36.5, 25.4, 25.2, 23.6, 17.4. ¹⁹F NMR (376 MHz, DMSO-d₆) *δ* = -63.48 (s, 3F). HRMS (ESI): m/z [M + H]⁺ calcd for: C₁₈H₂₇F₃NO₃: 362.1938; found: 362.1934.

### Example 4 Preparation of Compound 4:

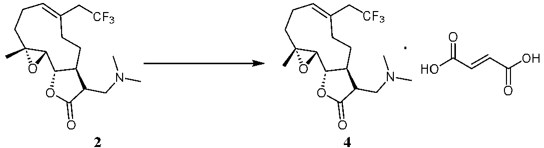

Compound **2** (361.2 mg, 1 mmol) and fumaric acid (116.1 mg, 1 mmol) were added to a round-bottom flask, followed by acetone (10 mL). The reaction was carried out at 50 °C for 0.5 h. The reaction mixture was concentrated to afford a white solid. Yield: 453.3 mg, 95%. Melting point: 213-214 °C. ¹H NMR (400 MHz, DMSO-*d*₆) *δ =* 6.60 (s, 2H), 5.56 (t, *J* = 8 Hz, 1H), 4.03 (t, *J* = 8 Hz, 1H), 3.24-3.07 (m, 1H), 2.97-2.90 (m, 1H), 2.75-2.66 (m, 4H), 2.47-2.22 (m, 8H), 2.17-2.01 (m, 3H), 1.71-1.65 (m, 1H), 1.47 (s, 3H), 1.02-0.87 (m, 1H). ¹³C NMR (100 MHz, DMSO-*d*₆): *δ* = 176.9, 166.4, 134.2, 132.6, 130.5, 126.8 (d, *J* = 277 Hz), 80.6, 63.2, 59.7, 57.4, 44.8, 42.7, 41.9, 37.5 (q, *J* = 28 Hz), 36.5, 25.3, 23.6, 17.4. ¹⁹F NMR (376 MHz, DMSO-*d*₆) *δ* = -63.53 (s, 3F). HRMS (ESI): m/z [M + H]⁺ calcd for: C₁₈H₂₇F₃NO₃: 362.1938; found: 362.1932.

### Example 5 Preparation of Compound 5:

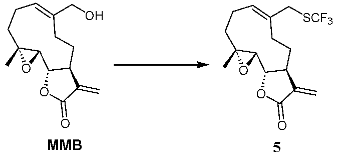

MMB (132.0 mg, 0.5 mmol), Ph₃P (183.6 mg, 0.7 mmol), and ICH₂CH₂I (197.3 mg, 0.7 mmol) were added to a Schlenk tube. Nitrogen was introduced, and then DMF (3 mL) was added. After stirring at room temperature until completely dissolved, ⁿBu₄N⁺I⁻ (277 mg, 0.75 mmol) and AgSCF₃ (314.9 mg, 1.5 mmol) dissolved in acetonitrile (1.5 mL) were added sequentially. The reaction mixture was stirred at 80 °C for 15 minutes. The reaction mixture was cooled to room temperature and filtered through a fritted funnel. The filtrate was concentrated, and the resulting crude product was purified on a silica gel column (petroleum ether:ethyl acetate = 8:1) to afford compound 1 as a white solid. Yield: 52 mg, 30 %. Melting point: 82-83 °C. ¹H NMR (400 MHz, CDCl₃): *δ =* 6.28 (d, *J =* 3.5 Hz, 1H), 5.70 (t, *J =* 8.1 Hz, 1H), 5.55 (d, *J* = 3.2 Hz, 1H), 3.85 (t, *J =* 9.3 Hz, 1H), 3.72 (d, *J =* 13.1 Hz, 1H), 3.41 (*d, J =* 13.1 Hz, 1H), 2.82 (d, *J =* 9.4 Hz, 1H), 2.78-2.64 (m, 1H), 2.57-2.37 (m, 2H), 2.37-2.10 (m, 4H), 1.78-1.67 (m, 1H), 1.56 (m, 3H), 1.11 (t, *J =* 12.3 Hz, 1H).¹³C NMR (100 MHz, CDCl₃): *δ* = 169.1, 138.5, 133.7, 131.5, 120.4, 80.9, 63.3, 59.9, 42.5, 36.6, 35.0, 25.0, 24.1, 23.6, 17.9;¹⁹F NMR (376 MHz, CDCl₃) *δ* = -41.13 (s, 3F). HRMS (ESI): m/z [M + H]⁺ calcd for: C₁₆H₂₀F₃O₃S: 349.1080; found: 349.1074.

### Example 6 Preparation of Compound 6:

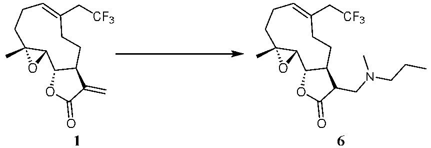

Compound **1** (316.1 mg, 1 mmol), N-methyl-n-propylamine (87.8 mg, 1.2 mmol), and potassium carbonate (165.9 mg, 1.2 mmol) were added to a round-bottom flask, followed by MeOH (10 mL). The reaction was carried out at room temperature for 2 h. The reaction mixture was filtered through a fritted funnel and then diluted with 50 mL of dichloromethane. The organic phase was washed with brine and dried over anhydrous Na2SO4. The crude product obtained by concentrating the organic phase was purified on a silica gel column (petroleum ether:ethyl acetate = 4:1) to afford the compound as a yellow oil. Yield: 268.5 mg, 69%. ¹H NMR (400 MHz, CDCl₃): *δ* = 5.60-5.49 (m, 1H), 3.83 (t, *J* = 9.6 Hz, 1H), 3.25-3.18 (m, 1H), 2.85-2.62 (m, 4H), 2.54-2.40 (m, 3H), 2.37-2.23 (m, 4H), 2.23-2.03 (m, 6H), 1.64-1.55 (m, 1H), 1.53 (s, 3H), 1.47 (m, 2H), 1.08 (t, *J =* 12.5 Hz, 1H), 0.87 (t, *J =* 7.4 Hz, 3H). ¹³C NMR (100 MHz, CDCl₃): *δ* = 176.9, 132.8, 130.9, 81.1, 63.8, 60.1, 59.8, 56.7, 44.0, 42.5, 41.8, 38.9 (q, *J=* 38.7 Hz), 36.8, 26.7, 25.6, 24.0, 20.0, 17.8, 14.1, 11.8. ¹⁹F NMR (376 MHz, CDCl₃): *δ* = -64.79 (s, 3F). HRMS (ESI): m/z [M + H]⁺ calcd for: C₂₀H₃₁F₃NO₃: 390.2251; found: 390.2247.

### Example 7: Preparation of Compound 7:

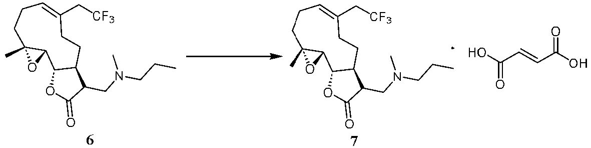

Compound 6 (389.2 mg, 1 mmol) and fumaric acid (116.1 mg, 1 mmol) were placed in a round-bottom flask, followed by the addition of acetone (10 mL). The reaction was carried out at 50 °C for 1 h. The reaction mixture was concentrated, and the resulting solid was washed with ethyl acetate and filtered to afford a white solid. Yield: 252.7 mg, 50%. Melting point: 187-188 °C. ¹H NMR (400 MHz, DMSO-*d*₆): *δ* = 6.59 (s, 2H), 5.60-5.53 (m, 1H), 4.01 (t, *J =* 9.6 Hz, 1H), 3.20-3.05 (m, 1H), 3.01-2.88 (m, 1H), 2.78-2.58 (m, 4H), 2.43-2.16 (m, 5H), 2.13 (s, 3H), 2.08 (m, 4H), 1.69-1.62 (m, 1H), 1.47 (s, 3H), 1.46-1.37 (m, 2H), 0.93 (dd, *J =* 20.9, 9.5 Hz, 1H), 0.83 (t, *J =* 7.4 Hz, 3H). ¹³C NMR (100 MHz, DMSO-*d*₆): δ = 177.2, 166.7, 134.4, 132.8, 130.4, 80.5, 63.1, 59.8, 59.6, 56.8, 42.9, 42.3, 41.5, 37.8 (q, *J* = 28 Hz), 36.5, 25.5 (d, *J =* 15 Hz), 23.6, 19.6, 17.4, 11.7. 1⁹F NMR (376 MHz, DMSO-d₆): δ = -63.56 (s, 3F). HRMS (ESI): m/z [M + H]⁺ calcd for: C₂₀H₃₁F₃NO₃: 390.2251; found: 390.2247.

### Example 8: Preparation of Compound 8:

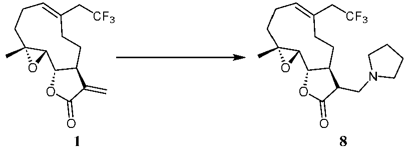

Compound **1** (316.1 mg, 1 mmol) and pyrrolidine (85.3 mg, 1.2 mmol) were placed in a round-bottom flask, followed by the addition of DCM (10 mL). The reaction was carried out at room temperature for 2 h. The reaction mixture was filtered through a fritted funnel and then diluted with 50 mL of dichloromethane. The organic phase was washed with brine and dried over anhydrous Na₂SO₄. The organic phase was concentrated, and the resulting crude product was purified on a silica gel column (petroleum ether: ethyl acetate = 4:1) to afford a white solid compound. Yield: 375.6 mg, 97%. Melting point: 172-173 °C. ¹H NMR (400 MHz, CDCl₃): *δ* = 5.53 (t, *J =* 8 Hz, 1H), 3.84 (t, *J* = 8 Hz, 1H), 3.29 (m, 1H), 2.99-2.87 (m, 1H), 2.81-2.76 (m, 1H), 2.71 (d, *J =* 9.3 Hz, 1H), 2.68-2.57 (m, 1H), 2.54-2.28 (m, 8H), 2.16 (m, 4H), 1.72 (s, 4H), 1.63-1.59 (m, 1H), 1.52 (s, 3H), 1.11-1.04 (m, 1H). ¹³C NMR (100 MHz, CDCl₃): *δ* = 176.8, 132.7, 131.1, 81.3, 64.0, 59.7, 54.1, 53.6, 44.6, 41.8, 38.8 (*q, J =* 29 Hz), 36.9, 26.4, 25.5, 24.0, 23.4, 17.8. ¹⁹F NMR (376 MHz, CDCl₃): *δ* = -64.83 (s, 3F). HRMS (ESI): m/z [M + H]⁺ calcd for: C₂₀H₂₉F₃NO₃: 388.2094; found: 388.2088.

### Example 9: Preparation of Compound 9:

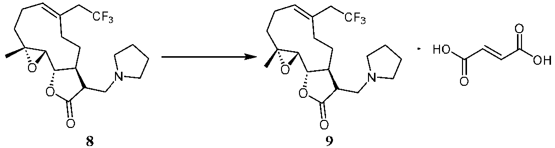

Compound **8** (387.2 mg, 1 mmol) and fumaric acid (116.1 mg, 1 mmol) were placed in a round-bottom flask, followed by the addition of acetone (10 mL). The reaction was carried out at 50 °C for 1 h. The reaction mixture was concentrated, and the resulting solid was washed with ethyl acetate and filtered to afford a white solid. Yield: 387.5 mg, 77%. Melting point: 216-217 °C. ¹H NMR (400 MHz, DMSO-*d*₆): *δ* = 6.59 (s, 2H), 5.59-5.53 (m, 1H), 4.04 (t, *J* = 9.6 Hz, 1H), 3.23-3.17 (m, 1H), 2.97-2.82 (m, 3H), 2.71 (dd, *J =* 11.9, 7.7 Hz, 2H), 2.59-2.54 (m, 4H), 2.43-2.18 (m, 3H), 2.15-2.02 (m, 4H), 1.73-1.68 (m, 5H), 1.47 (s, 3H), 1.04-0.87 (m, 1H). ¹³C NMR (100 MHz, DMSO-*d*₆): *δ* = 176.9, 166.6, 134.3, 132.7, 130.5, 80.7, 63.3, 59.7, 53.6, 53.0, 44.7, 43.6, 41.4, 37.7 (q, *J* = 27 Hz), 36.6, 25.2 (q, *J* = 5 Hz), 23.6, 23.0, 17.4. ¹⁹F NMR (376 MHz, DMSO-*d*₆): *δ =* -63.60. HRMS (ESI): m/z [M + H]⁺ calcd for: C₂₀H₂₉F₃NO₃: 388.2094; found: 388.2091.

### Example 10 Preparation of Compound 10:

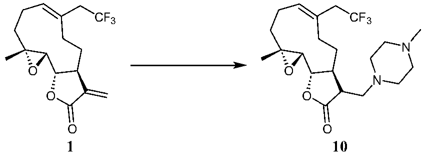

Compound **1** (316.1 mg, 1 mmol), N-methylpiperazine (120.2 mg, 1.2 mmol), and potassium carbonate (165.9 mg, 1.2 mmol) were added to a round-bottom flask, followed by MeOH (10 mL). The reaction was stirred at room temperature for 2 h. The reaction mixture was filtered through a fritted funnel and then diluted with 50 mL of dichloromethane. The organic phase was washed with brine and dried over anhydrous Na₂SO₄. The crude product obtained after concentration was purified on a silica gel column (petroleum ether: ethyl acetate = 1:1) to afford a white solid compound. Yield: 398.6 mg, 96%. Melting point: 110-112 °C. ¹H NMR (400 MHz, CDCl₃): *δ* = 5.65-5.51 (m, 1H), 3.86-3.81 (m, 1H), 3.32-3.18 (m, 1H), 2.85-2.80 (m, 1H), 2.76-2.68 (m, 2H), 2.66-2.61 (m, 1H), 2.59-2.27 (m, 11H), 2.25-2.19 (m, 3H), 2.19-2.03 (m, 4H), 1.64-1.54 (m, 1H), 1.52 (s, 3H), 1.12-1.04 (m, 1H). ¹³C NMR (100 MHz, CDCl₃): *δ* = 176.7, 133.0, 130.8, 81.1, 68.0, 63.8, 59.8, 57.5, 54.6, 46.6, 45.9, 43.1, 42.8, 39.7, 36.8, 26.8, 25.6, 24.0, 17.8. ¹⁹F NMR (376 MHz, CDCl₃): *δ* = -64.21 (s, 3F). HRMS (ESI): m/z [M + H]⁺ calcd for: C₂₁H₃₂F₃N₂O₃: 417.2360; found: 417.2352.

### Example 11 Preparation of Compound 11:

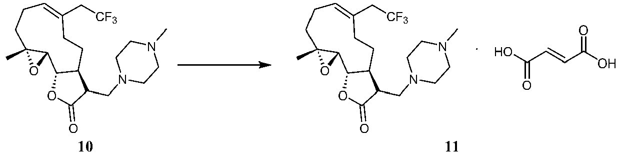

Compound **10** (416.2 mg, 1 mmol), and fumaric acid (116.1 mg, 1 mmol) were added to a round-bottom flask, followed by acetone (10 mL). The reaction was stirred at 50 °C for 1 h. The solid obtained after concentration was washed with ethyl acetate and filtered to afford a white solid. Yield: 319.4 mg, 60%. Melting point: 225-226 °C. ¹H NMR (400 MHz, DMSO-*d*₆): *δ* = 6.59 (s, 2H), 5.65-5.50 (m, 1H), 4.03 (t, *J= 9.5* Hz, 1H), 3.22-3.11 (m, 1H), 3.08-2.94 (m, 1H), 2.82-2.62 (m, 7H), 2.62-2.19 (m, 11H), 2.19-2.02 (m, 4H), 1.67 (t, *J* = 11.0 Hz, 1H), 1.48 (s, 3H), 0.95 (t, *J=* 12.3 Hz, 1H). ¹³C NMR (100 MHz, DMSO-d₆): δ = 177.6, 167.2, 134.9, 133.3, 130.9, 127.3 (q, *J=* 276 Hz) ,81.1, 63.6, 60.3, 56.4, 53.5, 51.4, 44.0, 42.9, 42.1, 38.5 (q, *J* = 10 Hz), 37.0, 26.0, 25.8, 24.1, 17.9. ¹⁹F NMR (376 MHz, DMSO-d₆): δ = -63.06 (s, 3F). HRMS (ESI): m/z [M + H]⁺ calcd for: C₂₁H₃₂F₃N₂O₃: 417.2360; found: 417.2357.

### Example 12 Preparation of Compound12:

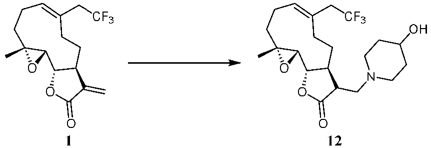

Compound **1** (316.1 mg, 1 mmol), 4-hydroxypiperidine (121.4 mg, 1.2 mmol), and potassium carbonate (165.9 mg, 1.2 mmol) were added to a round-bottom flask, followed by MeOH (10 mL). The reaction was stirred at room temperature for 2 h. The reaction mixture was filtered through a fritted funnel and then diluted with 50 mL of dichloromethane. The organic phase was washed with brine and dried over anhydrous Na₂SO₄. The organic layer was concentrated under reduced pressure to afford a white solid compound. Yield: 375.5 mg, 90%. Melting point: 123-124 °C. ¹H NMR (400 MHz, CDCl₃): *δ* = 5.56 (t, *J* = 8 Hz, 1H), 3.84 (t, *J* = 9.5 Hz, 1H), 3.73-3.63 (m, 1H), 3.35-3.12 (m, 1H), 2.85-2.71 (m, 4H), 2.66-2.57 (m, 2H), 2.51-2.43 (m, 3H), 2.37-2.06 (m, 8H), 1.91-1.81 (m, 2H), 1.75-1.67 (m, 1H), 1.62-1.59 (m, 1H), 1.54-1.53 (m, 3H), 1.51-1.46 (m, 1H), 1.08 (*t, J =* 12 Hz, 1H). ¹³C NMR (100 MHz, CDCl₃): *δ* = 176.9, 133.0, 130.8, 81.2, 63.8, 59.9, 57.2, 51.1, 46.6, 43.4, 42.6, 39.2 (q, *J=* 29 Hz), 36.8, 34.1, 34.0, 26.8, 25.6, 24.1, 17.9. ¹⁹F NMR (376 MHz, CDCl₃): *δ* = -64.40 (s, 3F). HRMS (ESI): m/z [M + H]⁺ calcd for: C₂₁H₃₁F₃NO₄: 418.2200; found: 418.2198.

### Example 13 Preparation of Compound 13:

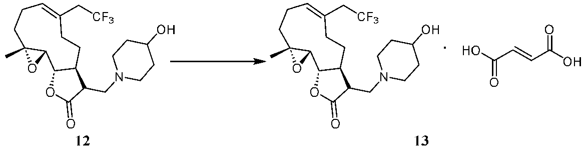

Compound **12** (417.2 mg, 1 mmol) and fumaric acid (116.1 mg, 1 mmol) were added to a round-bottom flask, followed by acetone (10 mL), and the mixture was reacted at 50 °C for 1 h. The reaction mixture was concentrated, and the resulting solid was washed with ethyl acetate and filtered to afford a white solid. Yield: 320.0 mg, 60%. Melting point: 198-199 °C. ¹H NMR (400 MHz, DMSO-*d*₆): *δ* = 6.59 (s, 2H), 5.58 (t, *J=* 8 Hz, 1H), 4.02 (t, *J=* 8 Hz, 1H), 3.47 (s, 1H), 3.27-3.21 (m, 1H), 3.01-2.89 (m, 1H), 2.74-2.70 (m, 3H), 2.65-2.64 (m, 3H), 2.41-2.27 (m, 3H), 2.21-1.91 (m, 7H), 1.75-1.65 (m, 3H), 1.47 (s, 3H), 1.43-1.28 (m, 2H), 0.95 (t, *J=* 12.3 Hz, 1H). ¹³C NMR (100 MHz, DMSO-*d*₆): *δ* = 177.3, 166.8, 134.5, 132.9, 130.6, 126.2 (d, *J* = 277 Hz), 80.6, 63.3, 59.9, 57.1, 50.7, 42.4, 42.2, 38.1 (q, *J* = 27 Hz), 36.5, 34.0, 33.8, 25.6, 25.4, 23.7, 17.5. ¹⁹F NMR (376 MHz, DMSO-*d*₆) *δ* = -63.28 (s, 3F). HRMS (ESI): m/z [M + H]⁺ calcd for: C₂₁H₃₁F₃NO₄: 418.2200; found: 418.2196.

### Example 14 Preparation of Compound14:

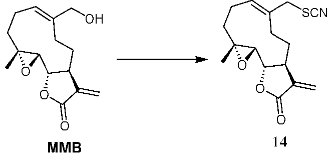

Into a Schlenk tube were added CuSCN (243.24 mg, 2.0 mmol), cesium fluoride (379.8 mg, 2.5 mmol), DMF (2 mL), and TMSCF₂H (186.3 mg, 1.5 mmol).The resulting mixture was stirred at 40°C for 60 min and then cooled to room temperature. MMB (132.0 mg, 0.5 mmol), Ph₃P (157.4 mg, 0.6 mmol), and ICH₂CH₂I (169.1 mg, 0.6 mmol) were added to a Schlenk tube. Nitrogen was introduced, and then DMF (3 mL) was added. After stirring at room temperature until complete dissolution, the mixture was added to the reaction mixture obtained in the previous step and stirred at room temperature for 12 hours. The reaction mixture was filtered through a fritted funnel. The filtrate was concentrated, and the resulting crude product was purified on a silica gel column (petroleum ether:ethyl acetate = 4:1) to afford compound **14** as a yellow solid. Yield: 45.8 mg, 30 %. Melting point: 155-157 °C. ¹H NMR (400 MHz, CDCl₃): *δ =* 6.24 (d, *J =* 4 Hz, 1H), 5.76 (t, *J* = 8 Hz, 1H), 5.56 (d, *J =* 4 Hz, 1H), 3.84 (t, *J =* 8 Hz, 1H), 3.76 (d, *J =* 12 Hz, 1H), 3.44 (d, *J =* 12 Hz, 1H), 2.82 (d, *J =* 12 Hz, 1H), 2.75-2.65 (m, 1H), 2.58-2.41 (m, 2H), 2.40-2.25 (m, 3H), 2.20-2.15 (m, 1H), 1.80-1.70 (m, 1H), 1.54 (s, 3H), 1.15-1.07 (m, 1H).¹³C NMR (100 MHz, CDCl₃): *δ* = 169.1, 138.3, 133.8, 133.3, 120.5, 111.4, 80.8, 63.1, 59.9, 42.3, 39.3, 36.4, 24.9, 24.1, 23.1, 17.9; HRMS (ESI): m/z [M + H]⁺ calcd for: C₁₆H₂₀NO₃S: 306.1158; found: 306.1158.

### Test Example 1 Anticancer Activity Test

Cells in logarithmic growth phase were digested, centrifuged, and counted; the cell suspension was adjusted to a concentration of 1500-3500 cells/well, and 95 µL was seeded into each well of a 96-well plate.

After continued overnight culture, drugs at different concentrations were added to the plate, with six replicate wells for each concentration. 10 µL of the drug was added to each well, followed by incubation for 72 h. Then, 10 µL of CCK-8 solution was added to each well (care should be taken to avoid generating bubbles in the wells, as they can affect the OD reading). After incubation in the incubator for 1-4 h, the OD value at 450 nm was measured using a microplate reader. The experiment to assess the effect of each drug on each cell line at different time points was repeated three times.

Data were processed using EXCEL software to calculate the inhibition rate at different concentrations, and SPSS was used to fit the IC50 value. EXCEL was used to calculate the mean and standard deviation of the three IC₅₀ values. Inhibition rate % = {1 - (OD value of drug well - OD value of blank well) / (OD value of control well - OD value of blank well) } * 100%.

**Table 1-1 Activity of Compounds in Inhibiting Cancer Cells**

| Compound | IC50 (µM) | | | | |
|---|---|---|---|---|---|
| | H520 | HT-29 | Huh-7 | U-118 | PANC-1 |
| PTL | 3.789±0.632 | 5.158±0.778 | 5.580±1.567 | 3.839±0.710 | 6.982±0.417 |
| Compound 1 | 1.791±0.552 | 3.309±1.014 | 1.890±0.312 | 1.823±0.357 | 2.259±0.954 |
| Compound 2 | 3.281±0.257 | 4.997±1.199 | 3.934±0.635 | 4.299±1.509 | 6.354±0.328 |
| Compound 3 | 4.490±0.197 | 5.999±1.190 | 5.462±0.654 | 5.025±1.317 | 8.802±0.780 |
| Compound 4 | 3.840±0.470 | 4.406±2.029 | 5.265±1.268 | 3.927±0.585 | 6.541±0.874 |
| Compound 5 | 2.125±0.485 | 2.930±1.003 | 2.903±0.805 | 3.151±0.759 | 2.230±0.576 |
| Compound 14 | 6.952±0.165 | 4.868±0.178 | 5.605±0.414 | 3.289±0.781 | 4.045±0.131 |

**Table 1-2 Activity of Compounds in Inhibiting PANC-1 Cells**

| Compound | IC50 (µM) |
|---|---|
| PTL | 6.156±0.141 |
| Compound 6 | 7.199±0.224 |
| Compound 7 | 5.059±0.619 |
| Compound 8 | 4.132±1.121 |
| Compound 9 | 6.574±1.732 |
| Compound 10 | 24.422±1.804 |
| Compound 11 | 16.772±4.203 |
| Compound 12 | 4.058±0.621 |
| Compound 13 | 3.865±0.095 |

| | |
|---|---|
| Note: PTL refers to parthenolide, and its structure is shown below: | |

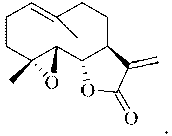

**Conclusion:** The compound exhibits certain efficacy against glioma, lung cancer, colon cancer, liver cancer, and pancreatic cancer.

### Test Example 2 Study on the Release of Compound 4 in Buffer Solution

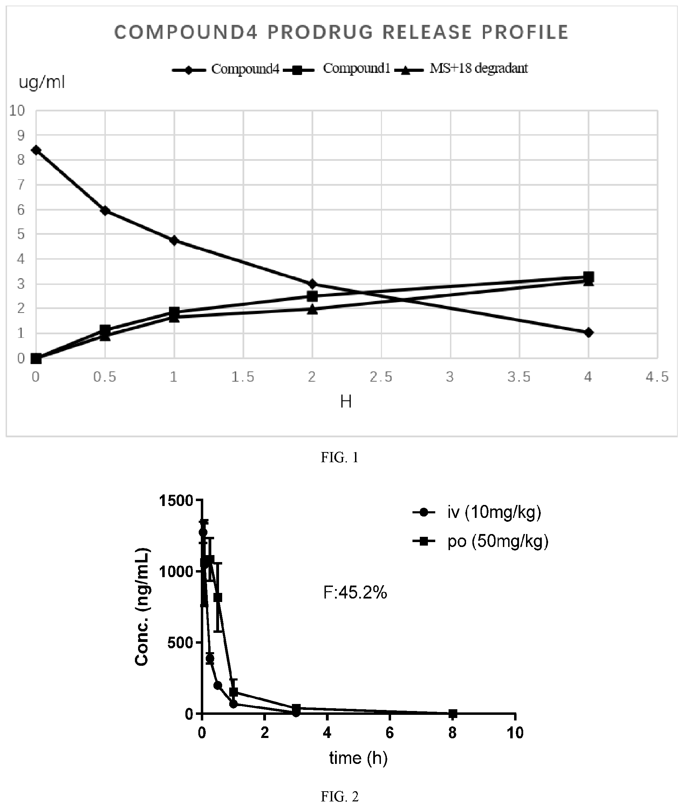

Compound 4 was taken and prepared into a test solution of approximately 10 µg/mL using a 0.01 M HEPES buffer solution (pH=7.4). The solution was placed in a 37°C water bath for incubation. At 0 h, 0.5 h, 1 h, 2 h, and 4 h, an appropriate amount of Compound 4 was taken out. The concentrations of Compound 4 and its degradation products were analyzed by HPLC to investigate the release rate of Compound 4. The results are shown in Figure 1.

**Conclusion:** In the buffer solution (pH=7.4), Compound 4 can slowly release the active substance, Compound 1.

### Test Example 3 Pharmacokinetic Study of Compound 4 and Its Distribution in Tissues Such as the Brain

(1) Forty-eight healthy Bar b/c mice, each weighing approximately 20 g, were taken and divided into 3 groups. Blank control group (6 mice), oral gavage administration group (Compound 4 dose: 50 mg/kg, 21 mice), and tail vein injection administration group (Compound 4 dose: 10 mg/kg, 21 mice)
(2) For the oral gavage group, blood samples were collected at 5 min, 15 min, 30 min, 1 h, 3 h, 8 h, and 24 h after administration. For the intravenous injection group, blood samples were collected at 2 min, 5 min, 15 min, 30 min, 1 h, 3 h, 8 h, and 24 h after administration. Blood was collected from mice via eyeball removal and placed in anticoagulant tubes containing sodium heparin. The samples were immediately centrifuged at 5000 rpm at low temperature (4°C) for 10 min to obtain plasma samples. The samples were stored in a -80°C freezer for analysis. Three mice were used per time point.
(3) The mouse was fixed on a foam board. The skin on the chest was lifted with forceps, and the thoracic skin and ribs were cut open with scissors to expose the heart and liver. The auricle was then cut open. A syringe needle was inserted into the left ventricle of the mouse, and normal saline was perfused for approximately 1 min (10-20 mL) until the mouse's limbs, liver, and tongue turned pale. Brain collection: The skin on the head was cut open to expose the white skull. The cartilage was cut, the skull cap was carefully pried open to expose the white brain, and the entire brain was dissected out. Tissues such as the pancreas, liver, lung, and rectum were also collected. The samples were stored in a -80°C freezer for analysis.
(4) The contents of Compound 4 and Compound 1 in peripheral blood and tissues were detected by LC/MS. MS/MS information

| Analyte | Q1 (m/z) | Q3 (m/z) | DP (v) | EP (v) | CE (v) | CXP (v) |
|---|---|---|---|---|---|---|
| Compound 4 | 362.2 | 58.0 | 95 | 10 | 60 | 15 |
| Compound 1 | 334.1 | 299.0 | 72 | 10 | 12 | 15 |
| Tolbutamide (IS) | 271.3 | 155.0 | 88 | 10 | 17 | 15 |

| | | | | | | |
|---|---|---|---|---|---|---|
| Note: Tolbutamide was used as the internal standard for calculating the peak area ratio. Compound 1 was quantified using the [M+NH⁻⁴]⁺ peak. | | | | | | |

The results are as follows:

**Table 2 Blood Concentrations (ng/mL) After a Single Oral Gavage and Intravenous Injection Administration of Compound 4**

| Time | iv (10 mg/kg) | | | | | po (50 mg/kg) | | | | |
|---|---|---|---|---|---|---|---|---|---|---|
| | 1 | 2 | 3 | Mean | SD | 1 | 2 | 3 | Mean | SD |
| 2 min | 1200 | 1270 | 1350 | 1273 | 75 | NS | NS | NS | NA | NA |
| 5min | 866 | 889 | 1380 | 1045 | 290 | 773 | 1040 | 1370 | 1061.0 | 299.1 |
| 15min | 369 | 366 | 431 | 389 | 37 | 1240 | 1070 | 940 | 1083.3 | 150.4 |
| 30min | 187 | 212 | 194 | 198 | 13 | 757 | 609 | 1080 | 815.3 | 240.9 |
| 1h | 56.2 | 48.2 | 100 | 68 | 28 | 254 | 105 | 101 | 153.3 | 87.2 |
| 3h | 3.32 | 12.9 | 6.07 | 7 | 5 | 29.6 | 66.7 | 18.6 | 38.3 | 25.2 |
| 8h | - | - | - | NA | NA | 0.189* | 0.335* | 0.623* | 0.4 | 0.2 |
| 24h | - | - | - | NA | NA | - | - | - | NA | NA |

| | | | | | | | | | | |
|---|---|---|---|---|---|---|---|---|---|---|
| Note: The lower limit of quantitation is 3 ng/mL; "-" indicates not evaluable; "*" indicates an estimated value; "NA" indicates not applicable. "NS" indicates no sample. | | | | | | | | | | |

**Table 3. Plasma Concentrations (ng/mL) of the Released Active Substance Compound 1 Following Single Oral Gavage and Intravenous Administration of Compound 4**

| Time | iv (10 mg/kg) | | | | | po (50 mg/kg) | | | | |
|---|---|---|---|---|---|---|---|---|---|---|
| | 1 | 2 | 3 | Mean | SD | 1 | 2 | 3 | Mean | SD |
| 2 min | 108 | 103 | 99.8 | 104 | 4.13 | NS | NS | NS | NA | NA |
| 5min | 74.1 | 68.2 | 168 | 103 | 56.0 | 89.6 | 80 | 133 | 101 | 28.2 |
| 15min | 36.1 | 42.0 | 38.8 | 39.0 | 2.95 | 177 | 148 | 143 | 156 | 18.4 |
| 30min | 16.8 | 21.1 | 17.6 | 18.5 | 2.29 | 102 | 116 | 154 | 124 | 26.9 |
| 1h | 11.7 | 12.5 | 16.7 | 13.6 | 2.69 | 65.1 | 52.4 | 40.6 | 52.7 | 12.3 |
| 3h | 2.18* | 2.95* | 6.48 | 3.87 | 2.29 | 24.0 | 21.7 | 19.4 | 21.7 | 2.30 |
| 8h | BLOQ | BLOQ | 0.766* | NA | NA | 2.33* | BLOQ | 3.99* | 3.16 | 1.17 |
| 24h | BLOQ | BLOQ | BLOQ | NA | NA | BLOQ | BLOQ | BLOQ | NA | NA |

| | | | | | | | | | | |
|---|---|---|---|---|---|---|---|---|---|---|
| Note: The lower limit of quantitation is 3 ng/mL; "-" indicates not evaluable; "*" indicates an estimated value; "NA" indicates not applicable. "NS" indicates no sample. | | | | | | | | | | |

**Table 4. Concentrations (ng/g) of Compound 4 in Brain Tissue Following Single Oral Gavage and Intravenous Administration**

| Time | iv (10 mg/kg) | | | | | po (50 mg/kg) | | | | |
|---|---|---|---|---|---|---|---|---|---|---|
| | 1 | 2 | 3 | Mean | SD | 1 | 2 | 3 | Mean | SD |
| 2 min | 2150 | 1700 | 2210 | 2020 | 279 | NS | NS | NS | NA | NA |
| 5min | 1540 | 464 | 1400 | 1135 | 585 | 1230 | 2260 | 1700 | 1730 | 516 |
| 15min | 407 | 1760 | 410 | 859 | 780 | 2450 | 2220 | 1470 | 2047 | 512 |
| 30min | 81.1 | 65.8 | 123 | 90.0 | 29.6 | 1040 | 763 | 1460 | 1088 | 351 |
| 1h | 249 | 278 | 232 | 253 | 23.3 | 377 | 149 | 124 | 217 | 139 |
| 3h | 2.23* | 9.43* | 3.38* | 5.01 | 3.87 | 24.7 | 87.9 | 14.6 | 42.4 | 39.7 |
| 8h | - | - | - | NA | NA | 0.462* | 4.51* | - | 2.5 | 2.9 |
| 24h | - | - | - | NA | NA | - | - | - | NA | NA |

| | | | | | | | | | | |
|---|---|---|---|---|---|---|---|---|---|---|
| Note: The lower limit of quantitation is 13.5 ng/g; "-" indicates not evaluable; "*" indicates an estimated value; "NA" indicates not applicable. "NS" indicates no sample. | | | | | | | | | | |

**Table 5. Concentrations (ng/g) of Compound 4 in Pancreatic Tissue Following Single Oral Gavage and Intravenous Administration**

| Time | iv (10 mg/kg) | | | | | po (50 mg/kg) | | | | |
|---|---|---|---|---|---|---|---|---|---|---|
| | 1 | 2 | 3 | Mean | SD | 1 | 2 | 3 | Mean | SD |
| 2 min | 6130 | 8060 | 5780 | 6657 | 1228 | NS | NS | NS | NA | NA |
| 5min | 3920 | 3480 | 6260 | 4553 | 1494 | 8960 | 5740 | 8990 | 7897 | 1868 |
| 15min | 1730 | 2320 | 2340 | 2130 | 347 | 4910 | 5730 | 5020 | 5220 | 445 |
| 30min | 938 | 1440 | 1750 | 1376 | 409.8 | 4340 | 2220 | 7690 | 4750 | 2758 |
| 1h | 439 | 359 | 669 | 489 | 160.9 | 1290 | 486 | 602 | 793 | 435 |
| 3h | 17.1 | 63.9 | 33.6 | 38.2 | 23.74 | 223 | 588 | 104 | 305 | 252 |
| 8h | - | - | - | NA | NA | - | 0.0244* | 8.24* | 4.13 | 5.81 |
| 24h | - | - | - | NA | NA | - | - | - | NA | NA |

| | | | | | | | | | | |
|---|---|---|---|---|---|---|---|---|---|---|
| Note: The lower limit of quantitation is 13.5 ng/g; "-" indicates not evaluable; "*" indicates an estimated value; "NA" indicates not applicable. "NS" indicates no sample. | | | | | | | | | | |

**Table 6. Concentrations (ng/g) of Compound 4 in the Rectum Following Single Oral Gavage and Intravenous Administration**

| Time | iv (10 mg/kg) | | | | | po (50 mg/kg) | | | | |
|---|---|---|---|---|---|---|---|---|---|---|
| | 1 | 2 | 3 | Mean | SD | 1 | 2 | 3 | Mean | SD |
| 2 min | 3110 | 3090 | 2450 | 2883 | 375 | NS | NS | NS | NA | NA |
| 5min | 4450 | 4120 | 3900 | 4157 | 277 | 2130 | 5040 | 3220 | 3463 | 1470 |
| 15min | 1660 | 2060 | 1620 | 1780 | 243 | 5970 | 2390 | 2170 | 3510 | 2133 |
| 30min | 804 | 1470 | 3250 | 1841 | 1265 | 5940 | 2960 | 5590 | 4830 | 1629 |
| 1h | 455 | 369 | 529 | 451 | 80.1 | 1250 | 630 | 422 | 767 | 431 |
| 3h | 20.9 | 73.3 | 67.4 | 53.9 | 28.7 | 230 | 2450 | 134 | 938 | 1310 |
| 8h | - | - | - | NA | NA | 0.0331* | 56.1* | 20.7 | 25.6 | 28.4 |
| 24h | - | - | - | NA | NA | - | - | - | NA | NA |

| | | | | | | | | | | |
|---|---|---|---|---|---|---|---|---|---|---|
| Note: The lower limit of quantitation is 13.5 ng/g; "-" indicates not evaluable; "*" indicates an estimated value; "NA" indicates not applicable. "NS" indicates no sample. | | | | | | | | | | |

**Table 7 Concentrations (ng/g) of Compound 4 in Liver Following Single Oral Gavage and Intravenous Administration**

| Time | iv (10 mg/kg) | | | | | po (50 mg/kg) | | | | |
|---|---|---|---|---|---|---|---|---|---|---|
| | 1 | 2 | 3 | Mean | SD | 1 | 2 | 3 | Mean | SD |
| 2 min | 4470 | 3830 | 2520 | 3607 | 994 | NS | NS | NS | NA | NA |
| 5min | 3140 | 2940 | 4400 | 3493 | 792 | 26900 | 16300 | 24700 | 22633 | 5594 |
| 15min | 1110 | 1470 | 1630 | 1403 | 266 | 9670 | 8820 | 6660 | 8383 | 1552 |
| 30min | 636 | 932 | 798 | 789 | 148 | 2750 | 5260 | 6730 | 4913 | 2013 |
| 1h | 221 | 245 | 387 | 284 | 89.7 | 1220 | 412 | 655 | 762 | 415 |
| 3h | 17.2 | 53.9 | 33.2 | 34.8 | 18.4 | 189 | 500 | 96.6 | 262 | 211 |
| 8h | 0.91* | - | - | NA | NA | 1.54* | 6.97* | 2.55 | 3.7 | 2.9 |
| 24h | - | - | - | NA | NA | - | 13 | - | 13 | NA |

| | | | | | | | | | | |
|---|---|---|---|---|---|---|---|---|---|---|
| Note: The lower limit of quantitation is 13.5 ng/g; "-" indicates not evaluable; "*" indicates an estimated value; "NA" indicates not applicable. "NS" indicates no sample. | | | | | | | | | | |

**Table 8. Concentrations (ng/g) in Lung after Single Oral Gavage and Intravenous Administration of Compound 4**

| Time | iv (10 mg/kg) | | | | | po (50 mg/kg) | | | | |
|---|---|---|---|---|---|---|---|---|---|---|
| | 1 | 2 | 3 | Mean | SD | 1 | 2 | 3 | Mean | SD |
| 2 min | 4010 | 685 | 2880 | 2525 | 1691 | NS | NS | NS | NA | NA |
| 5min | 841 | 2220 | 4060 | 2374 | 1615 | 3110 | 4400 | 4020 | 3843 | 663 |
| 15min | 564 | 1460 | 498 | 841 | 537 | 2720 | 3050 | 3810 | 3193 | 559 |
| 30min | 532 | 475 | 456 | 488 | 40 | 993 | 495 | 2760 | 1416 | 1190 |
| 1h | 168 | 47.5 | 149 | 122 | 64.8 | 1150 | 557 | 466 | 724 | 371 |
| 3h | 5.82* | 27.9 | 13.6 | 15.8 | 11.2 | 122 | 332 | 62.9 | 172 | 141 |
| 8h | - | - | - | NA | NA | - | - | - | NA | NA |
| 24h | - | - | - | NA | NA | - | - | - | NA | NA |

| | | | | | | | | | | |
|---|---|---|---|---|---|---|---|---|---|---|
| Note: The lower limit of quantitation is 13.5 ng/g; "-" indicates not evaluable; "*" indicates an estimated value; "NA" indicates not applicable. "NS" indicates no sample. | | | | | | | | | | |

**Table 9. Concentrations (ng/g) of Compound 1 in Brain Tissue Following Single Oral Gavage and Intravenous Administration of Compound 4**

| Time | iv (10 mg/kg) | | | | | po (50 mg/kg) | | | | |
|---|---|---|---|---|---|---|---|---|---|---|
| | 1 | 2 | 3 | Mean | SD | 1 | 2 | 3 | Mean | SD |
| 2 min | 356 | 271 | 422 | 350 | 76 | NS | NS | NS | NA | NA |
| 5min | 303 | 97 | 276 | 225 | 112 | 245 | 414 | 446 | 368 | 108 |
| 15min | 69.6 | 370 | 69.8 | 170 | 173 | 507 | 441 | 300 | 416 | 106 |
| 30min | 25.8 | 16.1* | 25.5 | 22.5 | 5.52 | 268 | 209 | 348 | 275 | 69.8 |
| 1h | 49.5 | 8.09* | 41.0 | 32.9 | 21.9 | 107 | 65.4 | 60.0 | 77.5 | 25.7 |
| 3h | - | 0.863* | - | 0.86 | NA | 19.0* | 4.23* | 9.65* | 11.0 | 7.47 |
| 8h | - | - | - | NA | NA | - | 12.9* | - | 12.9 | NA |
| 24h | - | - | - | NA | NA | - | - | - | NA | NA |

| | | | | | | | | | | |
|---|---|---|---|---|---|---|---|---|---|---|
| Note: The lower limit of quantitation is 20 ng/g; "-" indicates not evaluable; "*" indicates an estimated value; "NA" indicates not applicable. "NS" indicates no sample. | | | | | | | | | | |

**Table 10. Concentrations (ng/g) of Compound 1 in Pancreatic Tissue Following Single Oral Gavage and Intravenous Administration of Compound 4**

| Time | iv (10 mg/kg) | | | | | po (50 mg/kg) | | | | |
|---|---|---|---|---|---|---|---|---|---|---|
| | 1 | 2 | 3 | Mean | SD | 1 | 2 | 3 | Mean | SD |
| 2 min | 78.6 | 68.3 | 46.1 | 64.3 | 16.6 | NS | NS | NS | NA | NA |
| 5min | 23.2 | 27.6 | 79.2 | 43.3 | 31.1 | 158 | 61.1 | 180 | 133 | 63.3 |
| 15min | 9.82* | 18.5* | 22.4 | 16.9 | 6.44 | 73.6 | 97.4 | 98.4 | 89.8 | 14.0 |
| 30min | 5.60* | 15.2* | 14.2* | 11.67 | 5.28 | 83.8 | 50.8 | 255 | 130 | 110 |
| 1h | 0.942* | - | - | 0.942 | NA | 28.0 | 29.0 | 25.6 | 27.5 | 1.75 |
| 3h | - | - | - | NA | NA | 23.4 | 12.4* | - | 17.9 | 7.78 |
| 8h | - | - | - | NA | NA | - | - | - | NA | NA |
| 24h | - | - | - | NA | NA | - | - | - | NA | NA |

| | | | | | | | | | | |
|---|---|---|---|---|---|---|---|---|---|---|
| Note: The lower limit of quantitation is 20 ng/g; "-" indicates not evaluable; "*" indicates an estimated value; "NA" indicates not applicable. "NS" indicates no sample. | | | | | | | | | | |

**Table 11 Concentrations (ng/g) of Compound 1 in Rectum Following Single Oral Gavage and Intravenous Administration of Compound 4**

| Time | iv (10 mg/kg) | | | | | po (50 mg/kg) | | | | |
|---|---|---|---|---|---|---|---|---|---|---|
| | 1 | 2 | 3 | Mean | SD | 1 | 2 | 3 | Mean | SD |
| 2 min | 85.6 | 113 | 57.9 | 85.5 | 27.6 | NS | NS | NS | NA | NA |
| 5min | 143 | 79.1 | 80.8 | 101 | 36.4 | 80.3 | 101 | 107 | 96 | 14 |
| 15min | 35.0 | 54.0 | 55.1 | 48.0 | 11.3 | 132 | 49.8 | 53.3 | 78.4 | 46.5 |
| 30min | 12.5* | 38.9 | 99.4 | 50.27 | 44.55 | 271 | 81.8 | 178 | 177 | 94.6 |
| 1h | 11.0* | - | 5.37* | 8.19 | 3.98 | 36.6 | 31.1 | 14.3* | 27.3 | 11.6 |
| 3h | 0.874* | 0.305* | - | NA | NA | 14.6* | 273 | - | 144 | 183 |
| 8h | - | - | - | NA | NA | - | 16.8* | - | 16.8 | NA |
| 24h | - | - | - | NA | NA | 0.779* | 4.90* | 14.2* | NA | NA |

| | | | | | | | | | | |
|---|---|---|---|---|---|---|---|---|---|---|
| Note: The lower limit of quantitation is 20 ng/g; "-" indicates not evaluable; "*" indicates an estimated value; "NA" indicates not applicable. "NS" indicates no sample. | | | | | | | | | | |

**Table 12. Concentration (ng/g) of Compound 1 in Liver Following Single Oral Gavage and Intravenous Administration of Compound 4**

| Time | iv (10 mg/kg) | | | | | po (50 mg/kg) | | | | |
|---|---|---|---|---|---|---|---|---|---|---|
| | 1 | 2 | 3 | Mean | SD | 1 | 2 | 3 | Mean | SD |
| 2 min | 223 | 113 | 81.1 | 139.0 | 74.4 | NS | NS | NS | NA | NA |
| 5min | 101 | 72.6 | 179 | 117.5 | 55.09 | 1090 | 898 | 1390 | 1126.0 | 248.0 |
| 15min | 38 | 72.6 | 62.1 | 57.6 | 17.74 | 460.0 | 417.0 | 287 | 388.0 | 90.07 |
| 30min | 24.9 | 42.5 | 29.4 | 32.3 | 9.14 | 147 | 206 | 306 | 219.7 | 80.38 |
| 1h | 14.5* | 14.5* | 23.1 | 17.4 | 4.97 | 59.8 | 46 | 61.9 | 55.9 | 8.64 |
| 3h | 2.32* | - | 4.09* | 3.21 | 1.25 | 38.4 | 55.9 | 11.5* | 35.3 | 22.37 |
| 8h | - | - | - | NA | NA | - | - | 17.3* | 17.3 | NA |
| 24h | - | - | - | NA | NA | - | - | - | NA | NA |

| | | | | | | | | | | |
|---|---|---|---|---|---|---|---|---|---|---|
| Note: The lower limit of quantitation is 20 ng/g; "-" indicates not evaluable; "*" indicates an estimated value; "NA" indicates not applicable. "NS" indicates no sample. | | | | | | | | | | |

**Table 13. Concentration (ng/g) of Compound 1 in the Lung Following Single Oral Gavage and Intravenous Administration of Compound 4**

| Time | iv (10 mg/kg) | | | | | po (50 mg/kg) | | | | |
|---|---|---|---|---|---|---|---|---|---|---|
| | 1 | 2 | 3 | Mean | SD | 1 | 2 | 3 | Mean | SD |
| 2 min | 77.7 | 3.85 | 35.2 | 38.9 | 37.1 | NS | NS | NS | NA | NA |
| 5min | 3.79* | 34.8 | 70.0 | 36.2 | 33.1 | 55.2 | 78.7 | 83 | 72.3 | 15.0 |
| 15min | 8.23* | 35.1 | 2.39* | 15.24 | 17.45 | 6.42* | 12.3* | 80 | 32.9 | 40.9 |
| 30min | 5.76* | 6.21* | 2.23* | 4.73 | 2.18 | 18.1* | 14.7* | 51.1 | 28.0 | 20.1 |
| 1h | 5.39* | - | - | 5.39 | NA | 38.6 | 31.5 | 25.6 | 32 | 6.5 |
| 3h | - | - | - | NA | NA | 14.7* | 18.7* | 7.19* | 14 | 5.8 |
| 8h | - | - | - | NA | NA | - | - | - | NA | NA |
| 24h | - | - | - | NA | NA | - | - | - | NA | NA |

| | | | | | | | | | | |
|---|---|---|---|---|---|---|---|---|---|---|
| Note: The lower limit of quantitation is 20 ng/g; "-" indicates not evaluable; "*" indicates an estimated value; "NA" indicates not applicable. "NS" indicates no sample. | | | | | | | | | | |

**Table 14 Comparison of Exposure in Plasma and Tissues After Single Oral Gavage and Intravenous Administration of Compound 4**

| Compound | Plasma or Tissue | AUC₀₋ₜ (hr*ng/mL, hr*ng/g) | | Fold (AUCₜᵢₛₛᵤₑ/AUCₚₗₐₛₘₐ ) | |
|---|---|---|---|---|---|
| | | iv (10mg/kg) | po (50 mg/kg) | iv (10mg/kg) | po (50 mg/kg) |
| Concentration of Compound 4 | Plasma | 438 | 991 | - | - |
| | Brain | 791 | 1477 | 1.80 | 1.49 |
| | Pancreas | 2523 | 5925 | 5.76 | 5.98 |
| | Rectum | 2297 | 7281 | 5.24 | 7.35 |
| | Liver | 1568 | 8430 | 3.58 | 8.51 |
| | Lung | 933 | 3193 | 2.13 | 3.22 |
| Concentration of Compound 1 after administration of Compound 4 | Plasma | 53 | 241 | - | - |
| | Brain | 133 | 403 | 2.49 | 1.67 |
| | Pancreas | 17 | 136 | 0.32 | 0.56 |
| | Rectum | 47 | 675 | 0.88 | 2.80 |
| | Liver | 70 | 541 | 1.32 | 2.24 |
| | Lung | 13 | 80 | 0.24 | 0.33 |

The results show that the compounds of the present disclosure have a higher concentration distribution in various tissues (especially liver tissue).

The implementation of the technical solution of the present disclosure has been exemplarily described above. It should be understood that the protection scope of the present disclosure is not limited to the above-described implementations. Any modification, equivalent replacement, improvement, and the like made by a person skilled in the art without departing from the spirit and principle of the present disclosure shall fall within the protection scope of the claims of the present application.

## Claims

1. A compound of formula (I), a deuterated form, tautomer, stereoisomer, hydrate, solvate, pharmaceutically acceptable salt, or prodrug thereof: 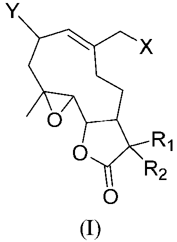 wherein:
X is selected from the following groups which are unsubstituted or optionally substituted with one, two, or more Rₓ: OH, SH, C₁₋₁₀ alkyl;
Y is selected from H, and the following groups which are unsubstituted or optionally substituted with one, two, or more R_{y}: OH, SH, C₁₋₁₀ alkyl;
each Rₓ and R_{y} is the same or different and is independently selected from H, OH, deuterium, halogen, CN, C₁₋₁₀ alkyl, C₁₋₁₀ alkoxy, halo-C₁₋₁₀ alkyl, halo-C₁₋₁₀ alkoxy, C₃₋₁₀ cycloalkyl, 3-10 membered heterocyclyl, C₆₋₁₄ aryl, 5-14 membered heteroaryl;
R₁ is selected from hydrogen or deuterium;
R₂ is selected from the following groups which is unsubstituted or optionally substituted with one, two, or more R₂₁: 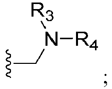 or 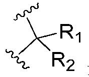 is selected from the following group which is unsubstituted or optionally substituted with one or two R₂₂: 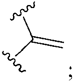
R₃ and R₄ are the same or different and are each independently selected from H, and the following groups which are unsubstituted or optionally substituted with one, two, or more R₃₁: C₁₋₁₀ alkyl, C₃₋₁₀ cycloalkyl, 3-10 membered heterocyclyl, C₆₋₁₄ aryl, 5-14 membered heteroaryl; or, R₃ and R₄ together with the N atom to which they are attached form a 3-10 membered heterocyclyl which is unsubstituted or optionally substituted with one, two, or more R₃₂;
each R₂₁, R₂₂, R₃₁, and R₃₂ is the same or different and is independently selected from H, OH, deuterium, halogen, CN, C₁₋₁₀ alkyl, C₃₋₁₀ cycloalkyl, 3-10 membered heterocyclyl, C₆₋₁₄ aryl, and 5-14 membered heteroaryl.

2. The compound, deuterated form, tautomer, stereoisomer, hydrate, solvate, pharmaceutically acceptable salt, or prodrug thereof according to claim 1, wherein X is selected from the following groups which are unsubstituted or optionally substituted with one, two, or more Rₓ: OH, SH, C₁₋₆ alkyl;
preferably, X is selected from the following groups which are unsubstituted or optionally substituted with one, two, or more Rₓ: OH, SH, methyl;
preferably, each Rₓ is the same or different and is independently selected from H, halogen, CN, C₁₋₆ alkyl, halo C₁₋₆ alkyl;
preferably, each Rₓ is the same or different and is independently selected from H, F, CN, CF₃;
preferably, X is selected from CF₃, OCF₃, SCF₃, SCN.

3. The compound, deuterated form, tautomer, stereoisomer, hydrate, solvate, pharmaceutically acceptable salt, or prodrug thereof according to claim 1 or 2, wherein Y is selected from H;
preferably, R₁ is hydrogen;
preferably, R₂ is selected from 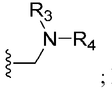
R₃ and R₄ are the same or different and are independently selected from H and C₁₋₆ alkyl; or, R₃ and R₄ together with the N to which they are attached form a 5- to 8-membered heterocyclic group which is unsubstituted or optionally substituted with one, two, or more R₃₂;
preferably, R₃ and R₄ are the same or different and are independently selected from H, methyl, ethyl, propyl; or, R₃ and R₄ together with the N to which they are attached form pyrrolidinyl, piperazinyl, or piperidinyl which is unsubstituted or optionally substituted with one, two, or more R₃₂;
preferably, each R₃₂ is the same or different and is independently selected from H, OH, and C₁₋₆ alkyl;
preferably, each R₃₂ is the same or different and is independently selected from H, OH, and methyl;
preferably, R₂ is selected from 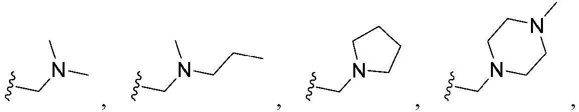 and 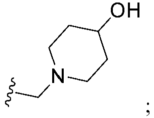
preferably,

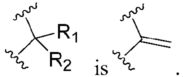

4. The compound, deuterated compound, tautomer, stereoisomer, hydrate, solvate, pharmaceutically acceptable salt, or prodrug thereof according to any one of claims 1-3, **characterized in that** the pharmaceutically acceptable salt is a pharmaceutically acceptable salt formed from the compound of formula (I) with an inorganic acid or an organic acid, including a quaternary ammonium salt formed from the compound of formula (I), wherein the inorganic acid or organic acid is selected from hydrofluoric acid, hydrochloric acid, hydrobromic acid, hydroiodic acid, sulfuric acid, phosphoric acid, nitric acid, phosphorous acid, sulfurous acid, carbonic acid, boric acid, phosphomolybdic acid, selenious acid, methanesulfonic acid, substituted methanesulfonic acid, benzenesulfonic acid, substituted benzenesulfonic acid, fumaric acid, citric acid, maleic acid, tartaric acid, oxalic acid, D-malic acid, L-malic acid, DL-malic acid, L-lactic acid, D-lactic acid, DL-lactic acid, formic acid, substituted formic acid, acetic acid, propionic acid, butyric acid, valeric acid, oleic acid, lauric acid, p-toluenesulfonic acid, 1-naphthalenesulfonic acid, 2-naphthalenesulfonic acid, phthalic acid, malonic acid, succinic acid, glycolic acid, thioglycolic acid, glycine, sarcosine, sulfonic acid, nicotinic acid, picolinic acid, isonicotinic acid, dichloroacetic acid, benzoic acid, and substituted benzoic acid.

5. The compound, deuterated compound, tautomer, stereoisomer, hydrate, solvate, pharmaceutically acceptable salt, or prodrug thereof according to any one of claims 1-4, **characterized in that** the compound of formula (I) has the following structure: 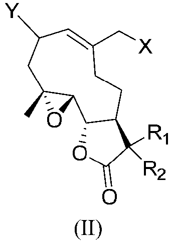
wherein X, Y, R₁, R₂ are as defined in any one of claims 1-4;
preferably, the compound of formula (I) has the following structure: 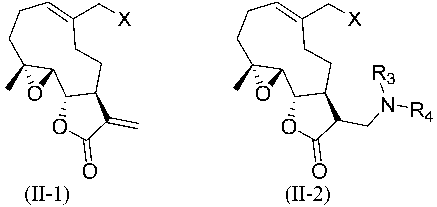
wherein X, R₃, R₄ are as defined in any one of claims 1-4.

6. The compound, deuterated compound, tautomer, stereoisomer, hydrate, solvate, pharmaceutically acceptable salt, or prodrug thereof according to any one of claims 1-5, **characterized in that** the compound of formula (I) is selected from the following structures: 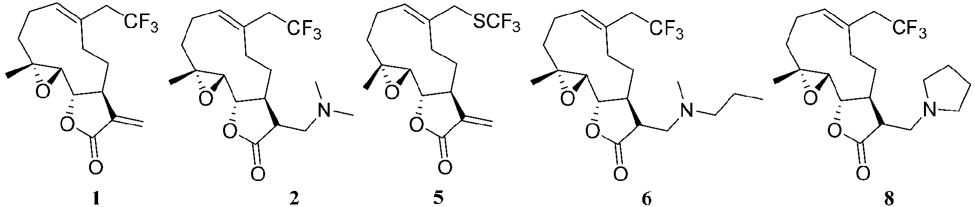 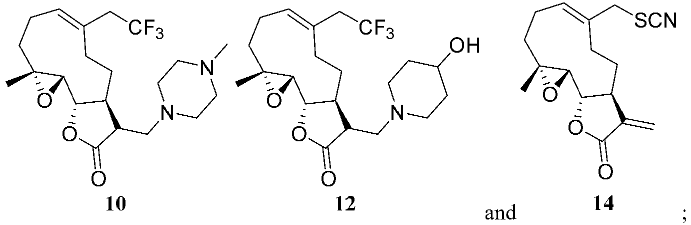 the pharmaceutically acceptable salt of the compound of formula (I) is selected from the following structures:

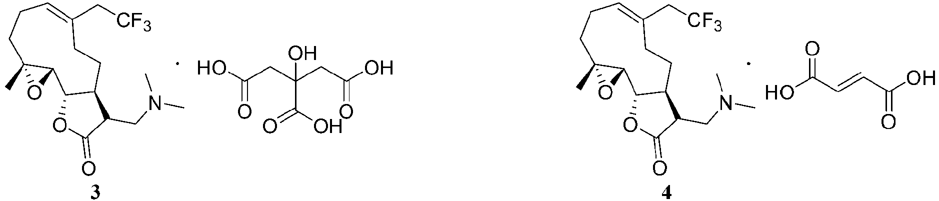

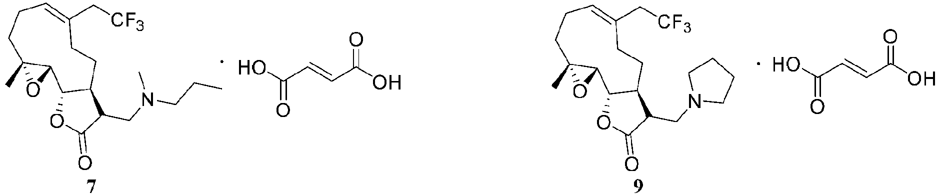

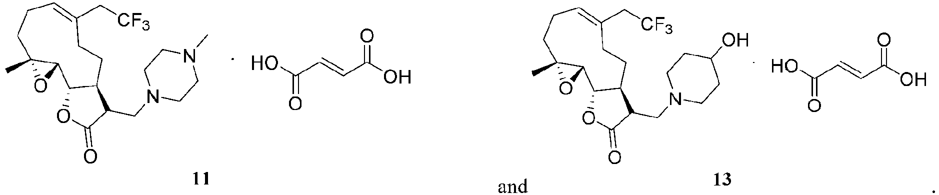

7. A method for preparing the compound according to any one of claims 1-6, a deuterated form, tautomer, stereoisomer, hydrate, solvate, pharmaceutically acceptable salt, or prodrug thereof, comprising the following steps: adding triphenylphosphine and a haloalkane to a solution of compound a, then adding Cu and a trifluoromethylating reagent to obtain a compound represented by formula (I); 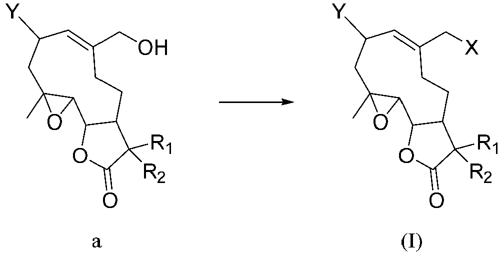
wherein X, Y, R₁, and R₂ are as defined in any one of claims 1-6;
the haloalkane is, for example, a dihaloalkane, preferably iodomethane, iodoethane, diiodomethane, or diiodoethane;
the trifluoromethylating reagent is, for example, methyl fluorosulfonyldifluoroacetate;
preferably, when 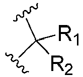 in formula (I) is 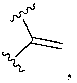 the preparation method further comprises the following step: reacting compound (I') with a compound NH(R₃)(R₄) to obtain compound (I"); 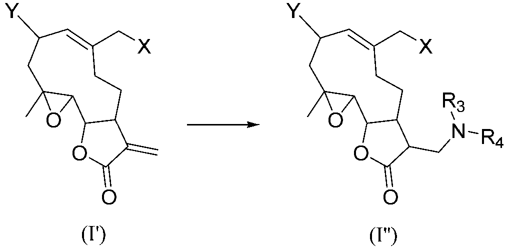
wherein X, Y, R₃, and R₄ are as defined in any one of claims 1-6; when 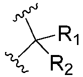 in formula (I) is 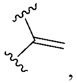 is denoted as (I'); when 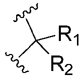 in formula (I) is 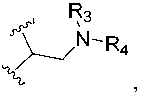 is denoted as (I").

8. The preparation method according to claim 7, **characterized by** comprising the following step: reacting the compound represented by formula (I) with an acid to obtain a pharmaceutically acceptable salt of the compound represented by formula (I);
the acid is selected from an inorganic acid or an organic acid, the inorganic acid or organic acid is selected from hydrofluoric acid, hydrochloric acid, hydrobromic acid, hydroiodic acid, sulfuric acid, phosphoric acid, nitric acid, phosphorous acid, sulfurous acid, carbonic acid, boric acid, phosphomolybdic acid, selenious acid, methanesulfonic acid, a substituted methanesulfonic acid, benzenesulfonic acid, a substituted benzenesulfonic acid, fumaric acid, citric acid, maleic acid, tartaric acid, oxalic acid, D-malic acid, L-malic acid, DL-malic acid, L-lactic acid, D-lactic acid, DL-lactic acid, formic acid, a substituted formic acid, acetic acid, propionic acid, butyric acid, valeric acid, oleic acid, lauric acid, p-toluenesulfonic acid, 1-naphthalenesulfonic acid, 2-naphthalenesulfonic acid, phthalic acid, malonic acid, succinic acid, glycolic acid, thioglycolic acid, glycine, sarcosine, sulfonic acid, nicotinic acid, picolinic acid, isonicotinic acid, dichloroacetic acid, benzoic acid, and a substituted benzoic acid.

9. A pharmaceutical composition, comprising the compound according to any one of claims 1-6, a deuterated form, tautomer, stereoisomer, hydrate, solvate, pharmaceutically acceptable salt, or prodrug thereof;
and/or, the pharmaceutical composition further comprises a pharmaceutically acceptable carrier;
and/or, the pharmaceutical composition further comprises another drug for treating cancer.

10. Use of the compound according to any one of claims 1-6, a deuterated form, tautomer, stereoisomer, hydrate, solvate, pharmaceutically acceptable salt, or prodrug thereof, or the pharmaceutical composition according to claim 9, in the manufacture of a medicament;
preferably, the medicament is a drug for treating cancer;
preferably, the cancer includes: leukemia, breast cancer, nasopharyngeal carcinoma, colorectal cancer, lung cancer, liver cancer, esophageal cancer, gastric cancer, intestinal cancer, kidney cancer, oral cancer, colorectal cancer, glioma, melanoma, bladder cancer, ovarian cancer, thyroid cancer, blood cancer, bone cancer, brain cancer, central nervous system cancer, cervical cancer, colon cancer, endometrial cancer, esophageal cancer, gallbladder cancer, gastrointestinal cancer, external genital cancer, genitourinary tract cancer, head cancer, laryngeal cancer, muscle tissue cancer, neck cancer, oral or nasal mucosal cancer, pancreatic cancer, prostate cancer, skin cancer, spleen cancer, small intestine cancer, testicular cancer.
